Europäisches Patentamt

European Patent Office

Office européen des brevets

(11) Publication number: **0 192 811 B1**

# EUROPEAN PATENT SPECIFICATION

(45) Date of publication of patent specification: **06.03.91**

(21) Application number: **85109295.7**

(22) Date of filing: **13.10.83**

(51) Int. Cl.⁵: **C12N 15/19**, C12P 21/02, C07K 13/00, C07K 15/26, C12N 15/24, C12N 15/10, A61K 37/02, A61K 37/66

(60) Publication number of the earlier application in accordance with Art.76 EPC: **0 109 748**

(54) **Cysteine-depleted muteins of biologically active proteins, their preparation, formulations containing them, and structural genes, vectors and organisms, and their production, suitable for use in the preparation of said muteins.**

(30) Priority: **19.10.82 US 435154**
**15.04.83 US 486162**

(43) Date of publication of application:
**03.09.86 Bulletin 86/36**

(45) Publication of the grant of the patent:
**06.03.91 Bulletin 91/10**

(84) Designated Contracting States:
**AT BE CH DE FR GB IT LI LU NL SE**

(56) References cited:
**EP-A- 128 467**
**EP-A- 136 489**
**EP-A- 0 109 748**
**WO-A-86/04606**

(73) Proprietor: **CETUS CORPORATION**
**1400 Fifty-Third Street**
**Emeryville California 94608(US)**

(72) Inventor: **Mark, David F.**
**217 Standbridge Ct.**
**Danville California 94526(US)**
Inventor: **Lin, Leo S.**
**40880 Los Pinos**
**Fremont California 94539(US)**
Inventor: **Yu Lu, Shi-Da**
**366 Euclid Avenue, No. 109**
**Oakland California 94610(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

NATURE, vol. 294, no. 5841, December 10, 1981 (New York, London); H.M. SHEPHARD et al.,pp. 563-565

## Description

This invention is in the general area of recombinant DNA technology. More specifically it relates to mutationally altered biologically active proteins that differ from their parent analogs by one or more substitutions of cysteine residues.

Biologically active proteins that are microbially produced via recombinant DNA (rDNA) technology may contain cysteine residues that are nonessential to their activity but are free to form undesirable intermolecular or intramolecular links. One such protein is microbially produced human beta interferon (IFN-$\beta$). In the course of the preparation of IFN-$\beta$ by rDNA techniques, it has been observed that dimers and oligomers of microbially produced IFN-$\beta$ are formed in E.coli extracts containing high concentrations of IFN-$\beta$. This multimer formation renders purification and separation of IFN-$\beta$ very laborious and time-consuming and necessitates several additional steps in purification and isolation procedures such as reducing the protein during purification and reoxidizing it to restore it to its original conformation, thereby increasing the possibility of incorrect disulfide bond formation. Furthermore, microbially produced IFN-$\beta$ has also been found to exhibit consistently low specific activity due perhaps to the formation of multimers or of random intramolecular disulfide bridges. It would be desirable, therefore, to be able to alter microbially produced biologically active proteins such as IFN-$\beta$ in a manner that does not affect their activity adversely but reduces or eliminates their ability to form intermolecular crosslinks or intramolecular bonds that cause the protein to adopt an undesirable tertiary structure (eg, a conformation that reduces the activity of the protein).

The present invention is directed to producing by directed mutagenesis techniques mutationally altered biologically active proteins (such proteins are called "muteins", Glossary of Genetics and Cytogenetics, 4th Ed, p 381, Springer-Verlag (1976)) that retain the activity of their parent analogs but lack the ability to form intermolecular links or undesirable intramolecular disulfide bonds. In this regard Shepard, H.M., et al, Nature (1981) 294 :563-565 describe a mutein of IFN-$\beta$ in which the cysteine at position 141 of its amino acid sequence (there are three cysteines in native human IFN-$\beta$ at positions 17, 31, and 141, Gene (1980) 10 :11-15 and Nature (1980) 285 :542-547) is replaced by tyrosine. This mutein was made by bacterial expression of a hybrid gene constructed from a partial IFN-$\beta$ cDNA clone having a G → A transition at nucleotide 485 of the IFN-$\beta$ gene. The mutein lacked the biological activity of native IFN-$\beta$ leading the authors to conclude that the replaced cysteine was essential to activity.

Directed mutagenesis techniques are well known and have been reviewed by Lather, R.F. and Lecoq, J.P. in Genetic Engineering Academic Press (1983) pp 31-50. Oligonucleotide-directed mutagenesis is specifically reviewed by Smith, M. and Gillam, S. in Genetic Engineering: Principles and Methods, Plenum Press (1981) 3 :1-32.

E.P.A. 136489 discloses muteins of interleukin-2 in which the cystein residue normally at position 125 is replaced by serine or alanine. However, this document is not relevant to a consideration of inventive step, only being citable under Article 54(3) EPC.

Aimilarly, E.P.A. 128467, whilst it discloses certain muteins of interferon-$\alpha$ and of hybrid interferon-$\alpha$s is also not relevant to inventive step, being citable only under Article 54(3) EPC.

Reference is also made to E.P.A. 0109748, from which the present case is divided, and which is specifically concerned with interleukin-2 mutein preparations.

The reader is also referred to:

(a) E.P.A. 0218825 which is concerned with interferon-beta muteins and which was also divided from E.P.A. 0109748; and

(b) E.P.A. 0234599 which is concerned with certain specific muteins (at position 125) of interleukin-2 and which was also divided from E.P.A. 0109748.

The present invention, however, provides a recombinant, synthetic biologically active mutein of a biologically active protein normally having at least one cysteine residue that is free to form a disulfide link and is non-essential to said biological activity in which at least one of said cysteine residues has been replaced by a neutral amino acid; provided that said mutein is not a serine$_{125}$interleukin-2 mutein, an alanine$_{125}$- interleukin-2 mutein, a threonine$_{125}$interleukin-2 mutein, a glycine$_{125}$interleukin-2 mutein, a valine$_{125}$interleukin-2 mutein, a leucine$_{125}$-interleukin-2 mutein, an isoleucine$_{125}$interleukin-2 mutein, a histidine$_{125}$interleukin-2 mutein, a tyrosine$_{125}$interleukin-2 mutein, a phenylalanine$_{125}$interleukin-2 mutein, a tryptophan$_{125}$interleukin-2 mutein, a methionine$_{125}$interleukin-2 mutein, a mutein of interferon-$\alpha$A in which the cysteine residue(s) at Position 1 and/or Position 98 has/have been replaced, a mutein of interferon-$\alpha$C to L in which the cysteine residue(s) at Position 1 and/or Position 99 has/have been replaced, a mutein of interferon-$\alpha$B in which the cysteine residue(s) at Position 1 and/or Position 100 has/have been replaced, a mutein of interferon-$\alpha$D or of a hybrid interferon-$\alpha$ containing an amino acid sequence of interferon-$\alpha$D

3

comprising the cysteine residue at Position 87 and in which the said cysteine residue has been replaced, or a mutein of a hybrid interferon-α in which the cysteine residue(s) at Position 1 and/or Position 98, 99 or 100 (as the case may be) has/have been replaced.

Another aspect of the invention is DNA sequences that have been specifically designed to encode the above described synthetic muteins. Other aspects of this aspect are expression vectors that include such sequences and their production, host cells or organisms transformed with such vectors and their production, and processes for making the synthetic mutein by culturing such transformants or their progeny retaining the ability to express said DNA sequence and recovering the mutein from the culture. In the case of muteins that have therapeutic utility, therapeutic formulations, whether for human or veterinary use (and optionally in unit dosage form) are another aspect of the invention.

Another aspect of the invention is a method of preventing a protein having at least one cysteine residue that is free to form a disulfide link from forming said link comprising mutationally altering the protein to bring about replacement of the cysteine residue with a neutral amino acid, e.g., with serine or threonine; provided that the resulting mutationally altered protein is not a serine$_{125}$interleukin-2 mutein, an alanine$_{125}$-interleukin-2 mutein, a threonine$_{125}$interleukin-2 mutein, a glycine$_{125}$interleukin-2 mutein, a valine$_{125}$interleukin-2 mutein, a leucine$_{125}$-interleukin-2 mutein, an isoleucine$_{125}$interleukin-2 mutein, a histidine$_{125}$interleukin-2 mutein, a tyrosine$_{125}$interleukin-2 mutein, a phenylalanine$_{125}$interleukin-2 mutein, a tryptophan$_{125}$interleukin-2 mutein, a methionine$_{125}$interleukin-2 mutein, a mutein of interferon-αA in which the cysteine residue(s) at Position 1 and/or Position 98 has/have been replaced, a mutein of interferon-αc to L in which the cysteine residue(s) at Position 1 and/or Position 99 has/have been replaced, a mutein of interferon-αB in which the cysteine residue(s) at Position 1 and/or Position 100 has/have been replaced, a mutein of interferon-αD or of a hybrid interferon-α containing an amino acid sequence of interferon-αD comprising the cysteine residue at Position 87 and in which the said cysteine residue has been replaced, or a mutein of a hybrid interferon-α in which the cysteine residue(s) at Position 1 and/or Position 98, 99 or 100 (as the case may be) has/have been replaced.

A further aspect of the invention is a method of preventing a protein having at least one cysteine residue that is free to form a disulfide link from forming said link in a case where said link can be inhibitory of biological activity in said protein, comprising employing site-specific mutagenesis to produce a vector encoding said protein modified by said cysteine residue being replaced, cloning said vector using it to transform a host, using the resulting transformed hosts or progeny thereof retaining the ability to express said modified protein to express protein encoded by the genotype thereof, assessing proteins thus expressed by comparative biological effectiveness analysis to determine the clone corresponding to the desired modified protein, and using said clone to produce said protein as a biologically effective entity.

Still another aspect of the invention is a method for making the above described DNA sequence by oligonucleotide-directed mutagenesis comprising the following steps:

(a) hybridizing single-stranded DNA comprising a DNA sequence that encodes the parent protein with a mutant oligonucleotide primer that is complementary to a region of the strand that includes the codon for the cysteine to be replaced or the antisense triplet paired with the codon, as the case may be, except for a mismatch with that codon or antisense triplet, as the case may be, that defines a triplet that encodes said neutral amino acid;

(b) extending the primer with DNA polymerase to form a mutational heteroduplex; and

(c) replicating the mutational heteroduplex.

The mutant oligonucleotide primers used in this process are another aspect of the invention. Such primers may be made by a process comprising synthesizing by a manner known per se a nucleotide sequence that is complementary to a region of the DNA sequence that includes the codon for the cysteine residue or the antisense triplet paired with said codon, as the case may be, except for a mismatch with said codon that defines a triplet that codes for a neutral amino acid.

The invention also provides:

(a) a synthetic mutein as defined above for use in prophylaxis, therapy or diagnosis practised on the human or animal body; and

(b) the use of a synthetic mutein as defined above in the preparation of a medicament.

The invention will now be further described and illustrated with reference to the accompanying drawings, in which:-

Figure 1 is a diagram of the amino acid sequence of IFN-β;

Figure 2 is a schematic illustration showing the preparation of a mutant IFN-β gene by oligonucleotide-directed mutagenesis;

Figure 3 shows a diagram of plasmid pβltrp including the IFN-β gene;

Figure 4 is a diagram of the cloning vector M13mp8 phage;

EP 0 192 811 B1

Figure 5 shows the restriction map of clone m13-β1;

Figure 6 shows the sequencing gel pattern of the mutant IFN-β$_{ser17}$ gene showing a single base change in the coding region;

Figure 7 is a diagram of the expression plasmid pTrp3;

Figure 9a shows the Hin fl restriction pattern of clone pSY2501 and Figure 8b shows the resulting two 169bp and 28bp fragments thereof;

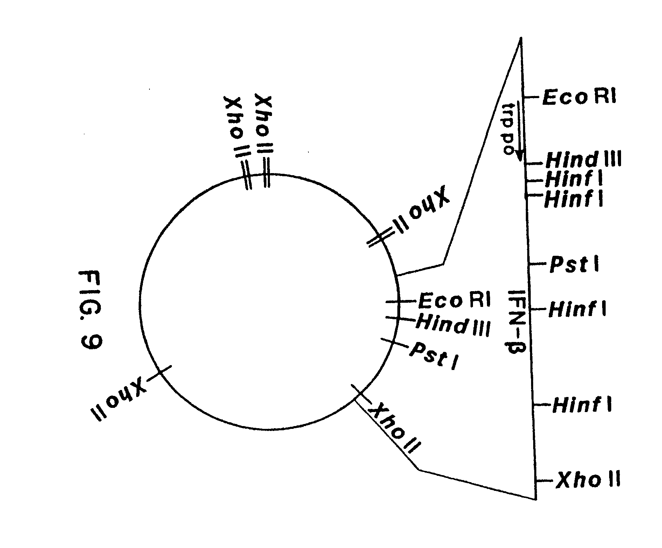

Figure 9 is a restriction map of clone pSY2501;

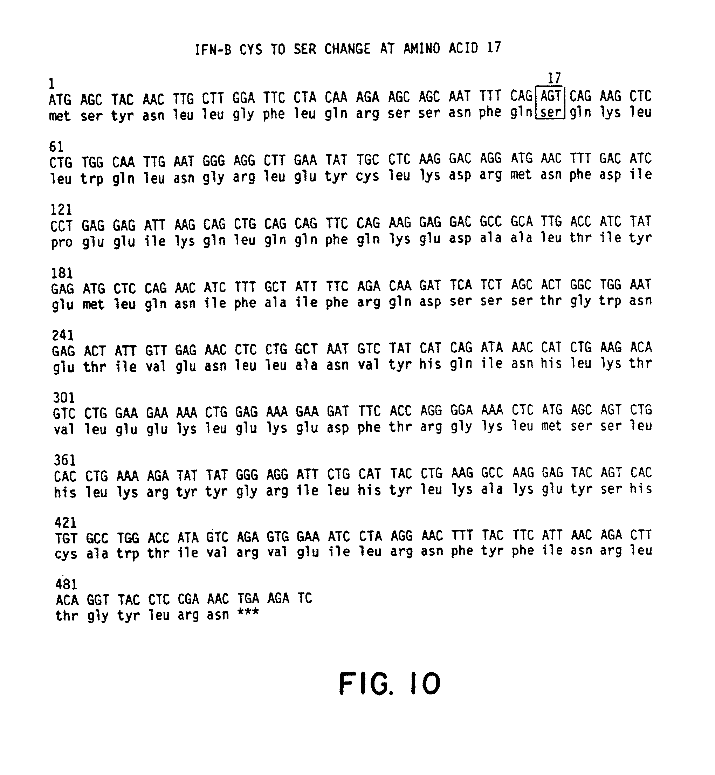

Figure 10 shows the coding DNA sequence for the mutein IFN-β$_{ser17}$ with the corresponding amino acid sequence therefor;

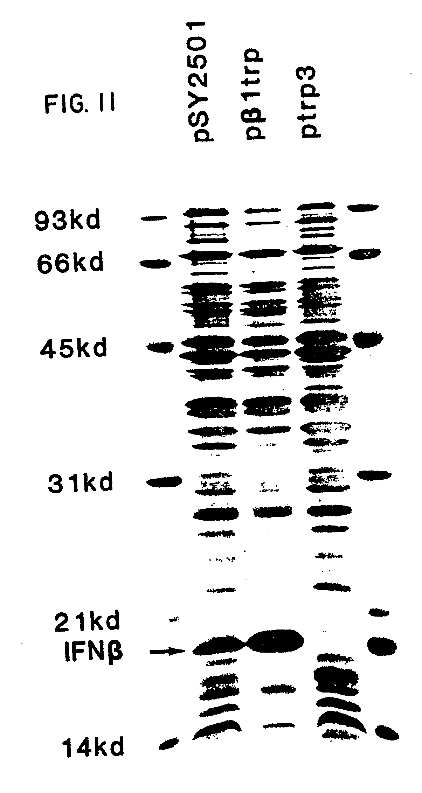

Figure 11 shows the single 18,000 dalton protein band corresponding to IFN-β$_{ser17}$ in the extracts of clones pSY2501 and pβltrp;

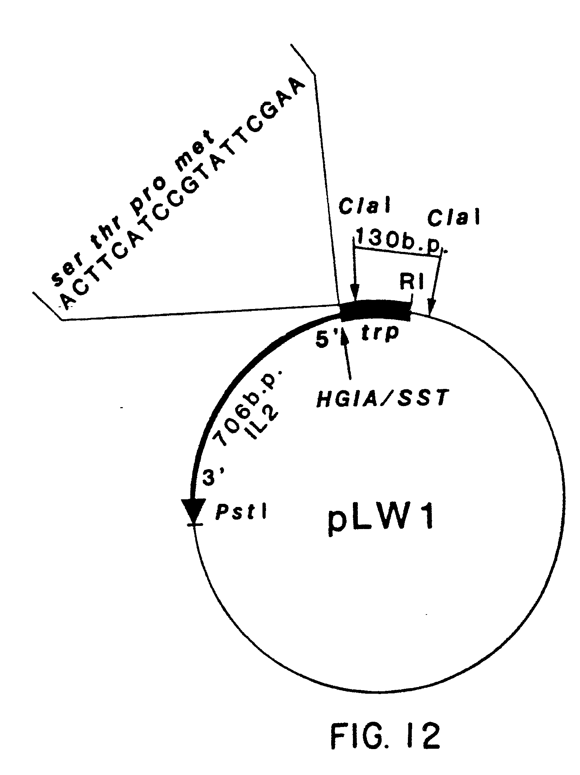

Figure 12 is a diagram of the plasmid pLW1 which contains the human interleukin-2 (IL-2) gene under the control of the E.coli trp promoter;

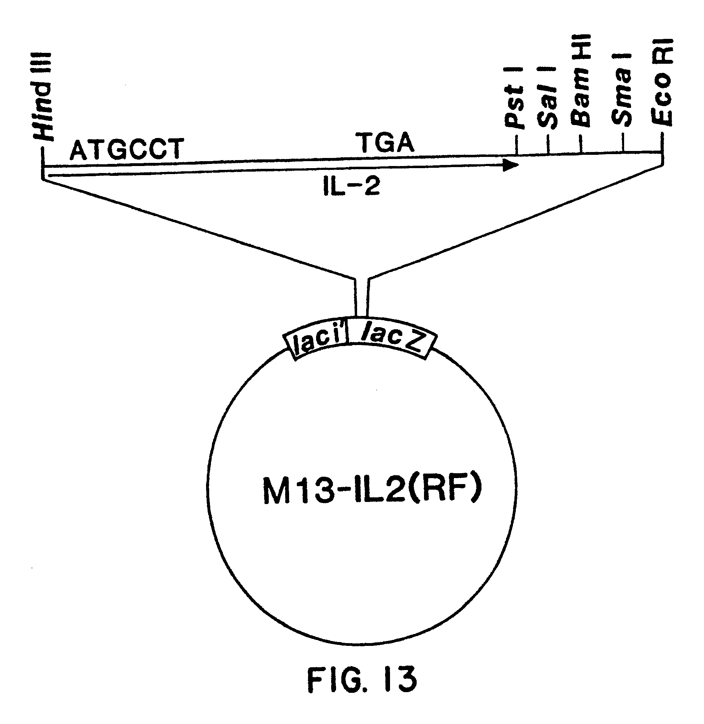

Figure 13 is a restriction map of phage clone M13-IL2;

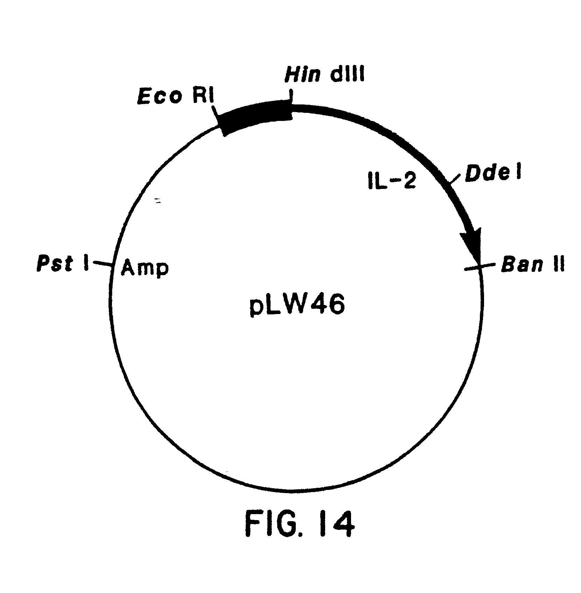

Figure 14 is a restriction map of the plasmid pLW46; and

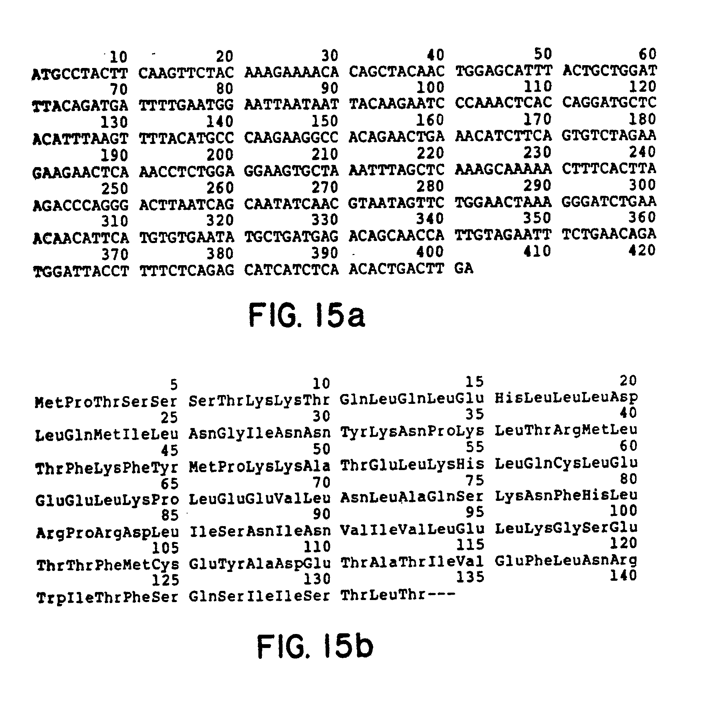

Figures 15a and 15b show, respectively, the nucleotide sequence of the coding strand of the clone pLW46 and the corresponding amino acid sequence of the IL-2 mutein designated IL-2$_{ser125}$.

The present invention provides: a certain category of muteins of biologically active proteins in which cysteine residues that are not essential to biological activity have been deliberately replaced with other amino acids to eliminate sites for intermolecular crosslinking or incorrect intramolecular disulfide bond formation; mutant sequences coding for such muteins; and means for making such muteins.

Proteins that may be mutationally altered according to this invention nay be identified from available information regarding the cysteine content of biologically active proteins and the roles played by the cysteine residues with respect to activity and tertiary structure. For proteins for which such information is not available in the literature this information may be determined by the skilled man by systematically altering each of the cysteine residues of the protein by the procedures described herein and testing the biological activity of the resulting muteins and their proclivity to form undesirable intermolecular or intramolecular disulfide bonds. Accordingly, while the nature of the invention is specifically illustrated below by reference to muteins of IFN-β and IL-2 it will be appreciated that the present invention is generally of applicability to any biologically active protein that contains a functionally non-essential cysteine residue that makes the protein susceptible to undesirable disulfide bond formation, and the specific teachings hereinafter are to be interpreted, accordingly, as clear guidance to the skilled reader in performing the generality of the present invention.

Examples of proteins other than IFN-β and IL-2 that are candidates for mutational alteration according to the invention are tumor necrosis factor and colony stimulating factor-1, and IFN-αl. Candidate proteins will usually have an odd number of cysteine residues. The invention includes, of course, muteins of such proteins.

In the case of IFN-β it has been reported in the literature that both the glycosylated and unglycosylated IFNs show qualitatively similar specific activities and that, therefore, the glycosyl moieties are not involved in and do not contribute to the biological activity of IFN-β. However, bacterially produced IFN-β which is unglycosylated consistently exhibits quantitatively lower specific activity than native IFN-β which is glycosylated. IFN-β is known to have three cysteine residues at positions 17, 31 and 141. Cysteine 141 has been demonstrated by Shepard, et al, supra, to be essential for biological activity. In IFN-α, which contains four cysteine residues, there are two intramolecular -S-S- bonds: one between cys 29 and cys 138 and another between cys 1 and cys 98. Based on the homology between IFN-β and IFN-αs cys 141 of IFN-β could be involved in an intramolecular -S-S- bond with cys 31, leaving cys 17 free to form intermolecular crosslinks. By either deleting cys 17 or substituting it by a different amino acid, one can determine whether cys 17 is essential to biological activity, and its role in -SS- bond formation. If cys 17 is not essential for the biological activity of the protein, the resulting cys 17-deleted or cys 17-substituted protein might exhibit specific activity close to that of native IFN-β and would possibly also facilitate isolation and purification of the protein.

By the use of the oligonucleotide-directed mutagenesis procedure with a synthetic oligonucleotide primer that is complementary to the region of the IFN-β gene at the codon for cys 17 but which contains single or multiple base changes in that codon, a designer gene may be produced that results in cys 17 being replaced with any other amino acid of choice. When deletion is desired the oligonucleotide primer lacks the codon for cys 17. Conversion of cys 17 to neutral amino acids such as glycine, valine, alanine, leucine, isoleucine, tyrosine, phenylalanine, histidine, tryptophan, serine, threonine and methionine is the preferred approach. Serine and threonine are the most preferred replacements because of their chemical

5

analogy to cysteine. When the cysteine is deleted, the mature mutein is one amino acid shorter than the native parent protein or the microbially produced IFN-$\beta$.

Human IL-2 is reported to have three cysteine residues located at positions 58, 105, and 125 of the protein. As in the case of IFN-$\beta$, IL-2 is in an aggregated oligomeric form when isolated from bacterial cells and has to be reduced with reducing agents in order to obtain a good yield from bacterial extracts. In addition, the purified reduced IL-2 protein is unstable and readily reoxidized upon storage to an oligomeric inactive form. The presence of three cysteines means that upon reoxidation, the protein may randomly form one of three possible intramolecular disulfide bridges, with only one of those being the correct bridge as found in the native molecule. Since the disulfide structure of the native IL-2 protein is not known, it is possible to

to create mutations at codons 58, 105 and 125 of the IL-2 gene and identify which cysteine residues are necessary for activity and therefore most likely to be involved in native disulfide bridge formation. In the same vein, the cysteine residue that is not necessary for activity can be modified so as to prevent the formation of incorrect intramolecular disulfide bridges and minimize the chance of intermolecular disulfide bridges by removal or replacement of the free cysteine residue.

The size of the oligonucleotide primer is determined by the requirement for stable hybridization of the primer to the region of the gene in which the mutation is to be induced, and by the limitations of the currently available methods for synthesizing oligonucleotides. The factors to be considered in designing oligonucleotides for use in oligonucleotide-directed mutagenesis (eg, overall size, size of portions flanking the mutation site) are described by Smith, M. and Gillam S., supra. In general the overall length of the oligonucleotide will be such as to optimize stable, unique hybridization at the mutation site with the 5' and 3' extensions from the mutation site being of sufficient size to avoid editing of the mutation by the exonuclease activity of the DNA polymerase. Oligonucleotides used for mutagenesis in accordance with the present invention usually contain from about 12 to about 24 bases, preferably from about 14 to about 20 bases and still more preferably from about 15 to about 18 bases. They will usually contain at least about three bases 3' of the altered or missing codon.

The method for preparing the modified IFN-$\beta$ gene broadly involves inducing a site-specific mutagenesis in the IFN-$\beta$ gene at codon 17 (TGT) using a synthetic nucleotide primer which omits the codon or alters it so that it codes for another amino acid. When threonine replaces the cysteine and the primer is hybridized to the antisense strand of the IFN-$\beta$ gene, the preferred nucleotide primer is GCAATTTTCAG ACT CAG (underlining denotes the altered codon). When it is desirable to delete cysteine, the preferred primer is AGCAATTTTCAGCAGAAGCTCCTG, which omits the TGT codon for cys. When cysteine is replaced by serine, a 17-nucleotide primer, GCAATTTTCAG AGT CAG, which includes an AGT codon for serine is the primer of choice. The T->A transition of the first base in the cys 17 codon results in changing cysteine to serine. It must be recognized that when deletions are introduced, the proper reading frame for the DNA sequence must be maintained for expression of the desired protein.

The primer is hybridized to single-stranded phage such as M13, fd, or ØX174 into which a strand of the IFN-$\beta$ gene has been cloned. It will be appreciated that the phage may carry either the sense strand or antisense strand of the gene. When the phage carries the antisense strand the primer is identical to the region of the sense strand that contains the codon to be mutated except for a mismatch with that codon that defines a deletion of the codon or a triplet that codes for another amino acid. When the phage carries the sense strand the primer is complementary to the region of the sense strand that contains the codon to be mutated except for an appropriate mismatch in the triplet that is paired with the codon to be deleted. Conditions that may be used in the hybridization are described by Smith, M. and Gillam, S., supra. The temperature will usually range between about 0°C and 70°C, more usually about 10°C to 50°C. After the hybridization, the primer is extended on the phage DNA by reaction with DNA polymerase I, T$_4$ DNA polymerase, reverse transcriptase or other suitable DNA polymerase. The resulting dsDNA is converted to closed circular dsDNA by treatment with a DNA ligase such as T$_4$ DNA ligase. DNA molecules containing single-stranded regions may be destroyed by S1 endonuclease treatment.

Oligonucleotide-directed mutagenesis may be similarly employed to make a mutant IL-2 gene that encodes a mutein having IL-2 activity but having cys 125 changed to serine 125. The preferred oligonucleotide primer used in making this mutant IL-2 gene when the phage carries the sense strand of the gene is GATGATGCTTCTG AGA AAAGGTAATC. This oligonucleotide has a C → G change at the middle base on the triplet that is paired with codon 125 of the IL-2 gene.

The resulting mutational heteroduplex is then used to transform a competent host organism or cell. Replication of the heteroduplex by the host provides progeny from both strands. Following replication the mutant gene may be isolated from progeny of the mutant strand, inserted into an appropriate expression vector, and the vector used to transform a suitable host organism or cell. Preferred vectors are plasmids

pBR322, pCRI, and variants thereof, synthetic vectors and the like. Suitable host organisms are E.coli, Pseudomonas, Bacillus subtilis, Bacillus thuringiensis, various strains of yeast, Bacillus thermophilus, animal cells such as mice, rat or Chinese hamster ovary (CHO) cells, plant cells and the like. It must be recognized that when a host of choice is transformed with the vector, appropriate promoter-operator sequences are also introduced in order for the mutein to be expressed. Hosts may be prokaryotic or eukaryotic (processes for inserting DNA into eukaryotic cells are described in PCT applications nos US81/00239 and US81/00240 published 3 September 1981). E.coli and CHO cells are the preferred hosts. The muteins obtained in accordance with the present invention may be glycosylated or unglycosylated depending on the glycosylation occurring in the native parent protein and the host organism used to produce the mutein. If desired, unglycosylated mutein obtained when E.coli or a Bacillus is the host organism, may be optionally glycosylated in vitro by chemical, enzymatic and other types of modifications known in the art.

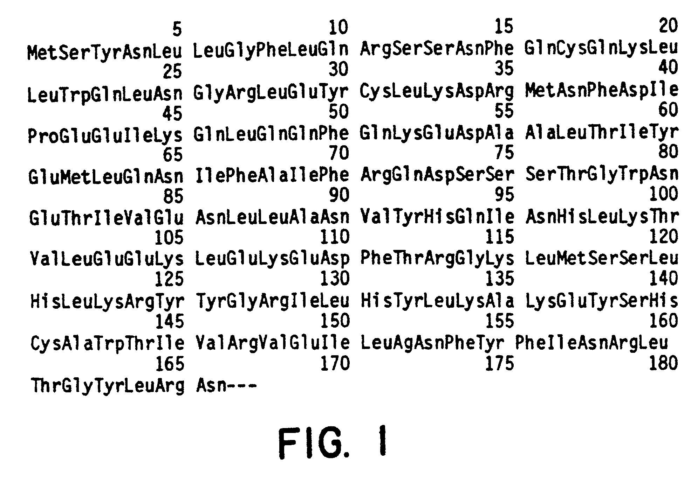

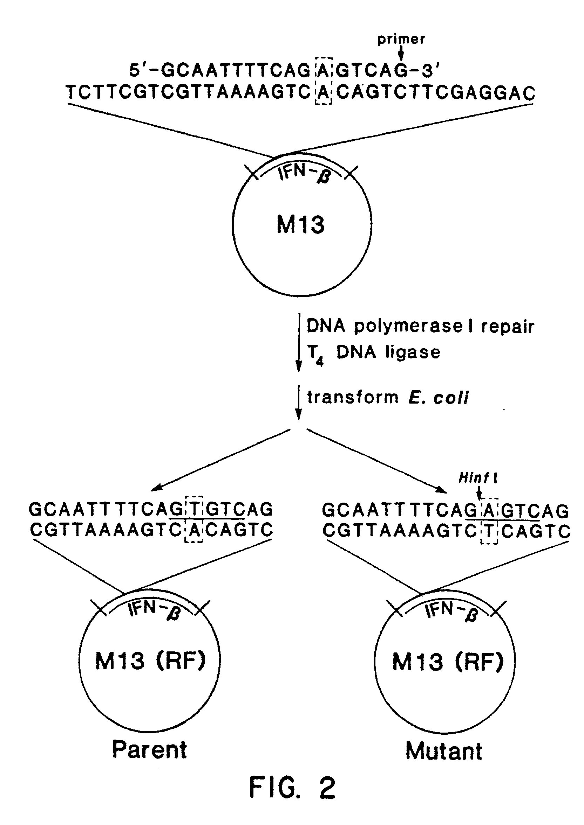

In connection with IFN-$\beta$, the cysteine residue at position 17 in the amino acid sequence of IFN-$\beta$, as shown in Figure 1, is changed to serine by a T → A transition of the first base of codon 17 of the sense strand of the DNA sequence which codes for the mature IFN-$\beta$. The site-specific mutagenesis is induced using a synthetic 17-nucleotide Primer GCAATTTTCAG AGT CAG which is identical to a seventeen nucleotide sequence on the sense strand of IFN-$\beta$ in the region of codon 17 except for a single base mismatch at the first base of codon 17. The mismatch is at nucleotide 12 in the primer. It must be recognized that the genetic code is degenerate and that many of the amino acids may be encoded by more than one codon. The base code for serine, for example, is six-way degenerate such that the codons, TCT, TCG, TCC, TCA, AGT, and ACG all code for serine. The AGT codon was chosen for the preferred embodiment for convenience. Similarly, threonine is encoded by any one of codons ACT, ACA, ACC and ACG. It is intended that when one codon is specified for a particular amino acid, it includes all degenerate codons which encode that amino acid. The 17-mer is hybridized to single-stranded M13 phage DNA which carrie's the antisense strand of the IFN-$\beta$ gene. The oligonucleotide primer is then extended on the DNA using DNA polymerase I Klenow fragment and the resulting dsDNA is converted to closed circular DNA with T$_4$ ligase. Replication of the resulting mutational heteroduplex yields clones from the DNA strand containing the mismatch. Mutant clones may be identified and screened by the appearance or disappearance of specific restriction sites, antibiotic resistance or sensitivity, or by other methods known in the art. When cysteine is substituted with serine, the T → A transition, shown in Figure 2, results in the creation of a new Hin fl restriction site in the structural gene. The mutant clone is identified by using the oligonucleotide primer as a probe in a hybridization screening of the mutated phage plaques. The primer will have a single mismatch when hybridized to the parent but will have a perfect match when hybridized to the mutated phage DNA, as indicated in Figure 2. Hybridization conditions can then be devised where the oligonucleotide primer will preferentially hybridize to the mutated DNA but not to the parent DNA. The newly generated Hin fl site also serves as a means of confirming the single base mutation in the IFN-$\beta$ gene.

The M13 phage DNA carrying the mutated gene is isolated and spliced into an appropriate expression vector, such as plasmid pTrp3, and E.coli strain MM294 is transformed with the vector. Suitable growth media for culturing the transformants and their progeny are known to those skilled in the art. The expressed mutein of IFN-$\beta$ is isolated, purified and characterized.

The following Examples are presented to help in the better understanding of the subject invention and for purposes of guidance. Examples 1-9 describe the preparation of a mutein of IFN-$\beta$. Examples 1-15 describe the preparation of a mutein of IL-2.

Example 1

Cloning of the IFN-$\beta$ Gene Into M13 Vector:

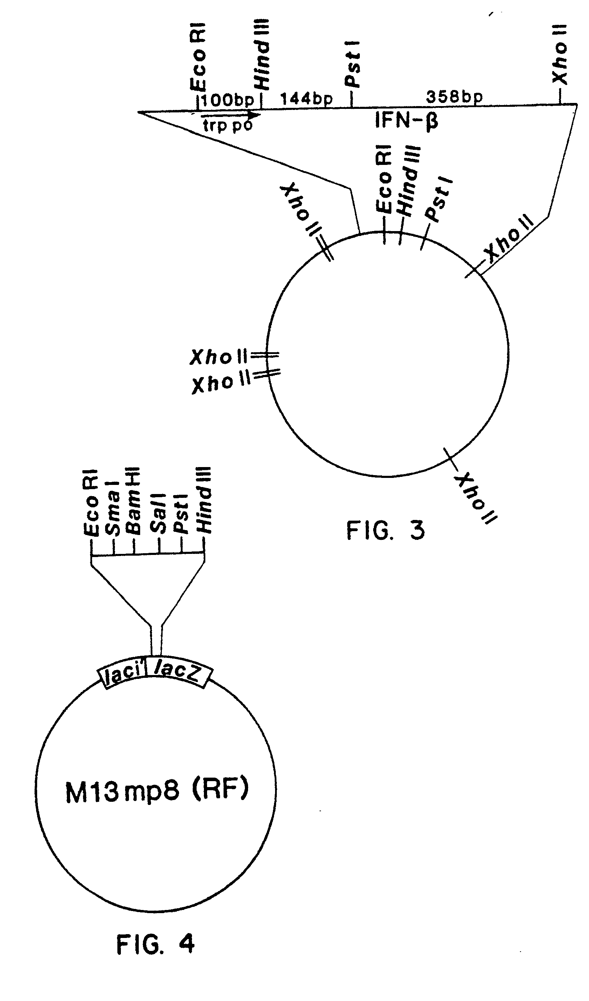

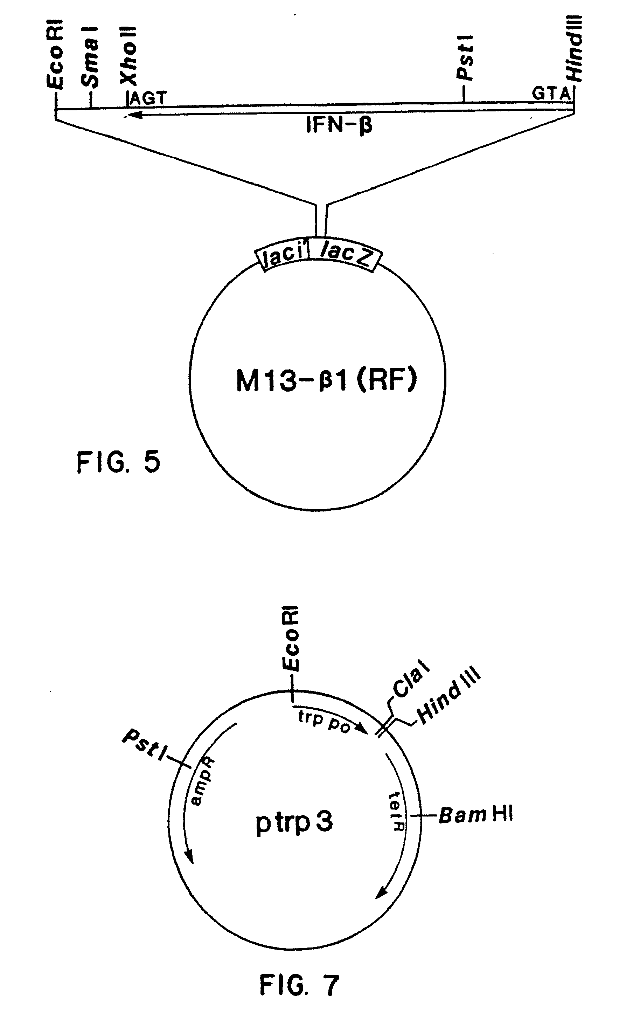

The use of M13 phage vector as a source of single-stranded DNA template has been demonstrated by G.F. Temple et al, Nature (1982) 296 :537-540. Plasmid p$\beta$ltrp (Figure 3) containing the IFN-$\beta$ gene under control of E.coli trp promoter, was digested with the restriction enzymes Hind III and Xho II. The M13mp8 (J. Messing, "Third Cleveland symposium on Macromolecules: Recombinant DNA," Ed. A Walton, Elsevier Press, 143-153 (1981)) replicative form (RF) DNA (Figure 4) was digested with restriction enzymes Hind III and Bam HI, and mixed with the p$\beta$ltrp DNA which had previously been digested with Hind III and Xho II. The mixture was then ligated with T$_4$ DNA ligase and the ligated DNA transformed into competent cells of E.coli strain JM 103 and plated on Xgal indicator plates (J. Messing, et al, Nucleic Acids Res (1981) 9 :309-321). Plaques containing recombinant phage (white plaques) were picked, inoculated into a fresh culture of JM 103 and minipreps of RF molecules prepared from the infected cells (H.D. Birnboim and J. Doly, Nucleic Acid Res (1979) 7 :1513-1523). The RF molecules were digested with various restriction enzymes to identify the clones containing the IFN-β insert. The restriction map of one such clone (M13-β1) is shown in Figure 5. Single-stranded (ss) phage DNA was prepared from clone M13-β1 to serve as a template for site-specific mutagenesis using a synthetic oligonucleotide.

Example 2

Site-Specific Mutagenesis:

Forty picomoles of the synthetic oligonucleotide GCAATTTTCAGAGTCAG (primer) was treated with $T_4$ kinase in the presence of 0.1 mM adenosine triphosphate (ATP), 50 mM hydroxymethylaminomethane hydrochloride (Tris-HCl) pH 8.0, 10 mM $MgCl_2$, 5 mM dithiothreitol (DTT) and 9 units of $T_4$ kinase, in 50 $\mu$l at 37°C for 1 hr. The kinased primer (12 pmole) was hybridized to 5 $\mu$g of ss M13-β1 DNA in 50 $\mu$l of a mixture containing 50 mM NaCl, 10 mM Tris-HCl, pH 8.0, 10 mM $MgCl_2$ and 10 mM β-mercaptoethanol, by heating at 67°C for 5 min and at 42°C for 25 min. The annealed mixture was then chilled on ice and then added to 50 $\mu$l of a reaction mixture containing 0.5 mM each of deoxynucleoside triphosphate (dNTP), 80 mM Tris-HCl, pH 7.4, 8 mM $MgCl_2$, 100 mM NaCl, 9 units of DNA polymerase I, Klenow fragment, 0.5 mM ATP and 2 units of $T_4$ DNA ligase, incubated at 37°C for 3 hr and at 25°C for 2 hr. The reaction was then terminated by phenol extraction and ethanol precipitation. The DNA was dissolved in 10 mM Tris-HCl 8.0, 10 mM ethylenediaminetetraacetic acid (EDTA), 50% sucrose and 0.05% bromophenylblue and electrophoresed on 0.8% agarose gel in the presence of 2 $\mu$g/ml of ethidium bromide. The DNA bands corresponding to the RF forms of M13 β1 were eluted from gel slices by the perchlorate method (R.W. Davis, et al, "Advanced Bacterial Genetics", Cold Spring Harbor Laboratory, N.Y., p. 178-179 (1980)). The eluted DNA was used to transform competent JM 103 cells, grown overnight and ssDNA isolated from the culture supernatant. This ssDNA was used as a template in a second cycle of primer extension, the gel purified RF forms of the DNA were transformed into competent JM 103 cells, plated onto agar plates and incubated overnight to obtain phage plaques.

Example 3

Site Specific Mutagenesis:

The experiment of Example 2 above is repeated except that the synthetic oligonucleotide primer used is GCAATTTTCAGACTCAG to change codon 17 of the IFN-β gene from one that codes for cysteine to one that codes for threonine.

Example 4

Site Specific Deletion:

The experiment of Example 2 above is repeated except that the synthetic oligonucleotide primer used is AGCAATTTTCAGCAGAAGCTCCTG to delete codon 17 of the IFN-β gene.

Example 5

Screening And Identification of Mutagenized Plaques:

Plates containing mutated M13-β1 plaques (Example 1) as well as two plates containing unmutated

EP 0 192 811 B1

M13-$\beta$1 phage plaques, were chilled to 4°C and plaques from each plate transferred onto two nitrocellulose filter circles by layering a dry filter on the agar plate for 5 min for the first filter and 15 min for the second filter. The filters were then placed on thick filter papers soaked in 0.2 N NaOH, 1.5 M NaCl and 0.2% Triton X-100 for 5 min, and neutralized by layering onto filter papers soaked with 0.5 M Tris-HCl, pH 7.5 and 1.5 M NaCl for another 5 min. The filters were washed in a similar fashion twice on filters soaked in 2 x SSC (standard saline citrate), dried and then baked in a vacuum oven at 80°C for 2 hr. The duplicate filters were prehybridized at 55°C for 4 hr with 10 ml per filter of DNA hybridization buffer (5 x SSC) pH 7.0, 4 x Denhardt's solution (polyvinylpyrrolidine, ficoll and bovine serum albumin, 1 x = 0.02% of each), 0.1% sodium dodecyl sulfate (SDS), 50 mM sodium phosphate buffer pH 7.0 and 100 $\mu$g/ml of denatured salmon sperm DNA. $^{32}$P-labeled probe was prepared by kinasing the oligonucleotide primer with $^{32}$P-labeled ATP. The filters were hybridized to 3.5 x 10$^5$ cpm/ml of $^{32}$P-labeled primer in 5 ml per filter of DNA hybridization buffer at 55°C for 24 hr. The filters were washed at 55°C for 30 min each in washing buffers containing 0.1% SDS and decreasing amounts of SSC. The filters were washed initially with buffer containing 2 x SSC and the control filters containing unmutated M13-$\beta$1 plaques were checked for the presence of any radioactivity using a Geiger counter. The concentration of SSC was lowered stepwise and the filters washed until no detectable radioactivity remained on the control filters with the unmutated M13-$\beta$1 plaques. The lowest concentration of SSC used was 0.1 x SSC. The filters were air dried and autoradiographed at -70°C for 2-3 days. 480 plaques of mutated M13-$\beta$1 and 100 unmutated control plaques were screened with the kinased oligonucleotide probe. None of the control plaques hybridized with the probe while 5 mutated M13-$\beta$1 plaques hybridized with the probe.

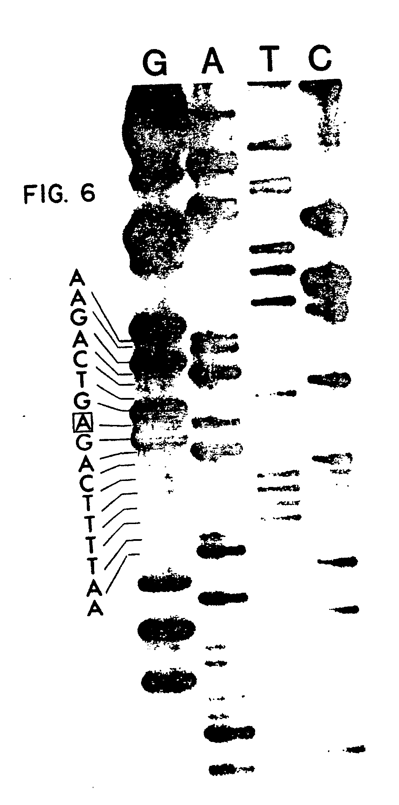

One of the five mutated M13-$\beta$1 plaques (M13-SY2501) was picked and inoculated into a culture of JM 103. ssDNA was prepared from the supernatant and double-stranded (ds) DNA was prepared from the cell pellet. The ssDNA was used as a template for the dideoxy-sequencing of the clone using the M13 univer sal primer. The result of the sequence analysis is shown in Figure 6, confirming that the TGT cys codon has been converted to an AGT ser codon.

Example 6

Expression of Mutated IFN-$\beta$ in E.coli:

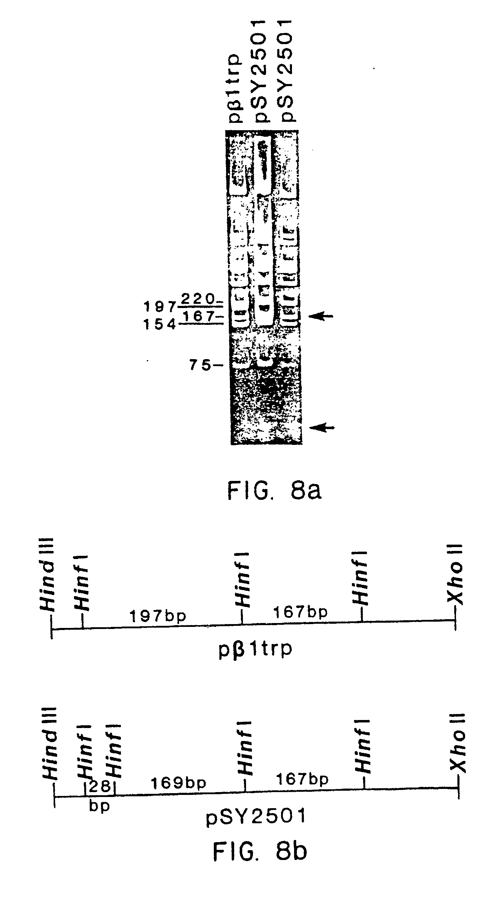

RF DNA from M13-SY2501 was digested with restriction enzymes Hind III and Xho II and the 520 bp insert fragment purified on a 1% agarose gel. The plasmid pTrp3 containing the E.coli trp promoter (Figure 7) was digested with the enzymes Hind III and Bam HI, mixed with the purified M13-SY250l DNA fragment, and ligated in the presence of T$_4$ DNA ligase. The ligated DNA was transformed into E.coli strain MM294. Ampicillin resistant transformants were screened for sensitivity to the drug tetracycline. Plasmid DNA from five ampicillin resistant, tetracylcine sensitive clones were digested with Hin fI to screen for the presence of the M13-SY2501 insert. Figure 8a shows the Hin fI restriction pattern of one of the clones (pSY2501), comparing it with the Hin fI pattern of the original IFN-$\beta$ clone, p$\beta$ltrp. As expected, there is an additional Hin fI site in pSY2501, cleaving the 197 bp IFN-$\beta$ internal fragment to a 169 bp fragment and a 28 bp fragment (Figure 8b). A restriction map of the clone pSY2501 is shown in Figure 9. The complete DNA sequence of the mutant IFN-$\beta$ gene is shown in Figure 10 together with the predicted amino acid sequence.

The plasmid designated as clone pSY2501 was deposited on 30 March 1933 with the Agricultural Research Culture Collection (NRRL), Fermentation Laboratory, Northern Regional Research Center, Science and Education Administration, U.S. Department of Agriculture, 1815 North University Street, Peoria, Illinois 60604 and is assigned accession numbers CMCC No. 1533 and NRRL No. B-15356.

Cultures of pSY2501 and p$\beta$ltrp, which include progeny thereof, were grown up to an optical density (OD$_{600}$) of 1.0. Cell free extracts were prepared and the amount of IFN-$\beta$ antiviral activity assayed on GM2767 cells in a microtiter assay. Extracts of clone pSY2501 exhihited three to ten times higher activity than p$\beta$ltrp (Table I), indicating that clone pSY2501 was either synthesizing more protein exhibiting IFN-$\beta$ activity or that the protein made had a higher specific activity.

9

## Table I

| EXTRACT | ANTIVIRAL ACTIVITY (U/ml) |
|---------|---------------------------|
| pSY2501 | $6 \times 10^5$ |
| pβltrp | $1 \times 10^5$ |
| ptrp3 (control) | 30 |

In order to determine if clone pSY2501 was synthesizing several times more active protein, the extracts of both clones were electrophoresed on a SDS polyacrylamide gel together with a control extract and the gel stained with coomasie blue to visualize the proteins. As shown in Figure 11, there was only one protein band corresponding to an apparent 18,000 dalton protein that was present in the extracts of clones pSY2501 and pβltrp but not in the control extract of ptrp3. This protein, which has a molecular weight of about 20,000 daltons but shows a gel migration pattern of an 18,000 dalton protein was previously shown to be IFN-$\beta$ by purification of this protein from extracts of pβltrp. Since there is less of this protein in extracts of pSY2501 than in extracts of pβltrp, the specific activity of the protein in extracts of clone pSY2501 was higher than that of clone pβltrp.

Example 7

Purification of IFN-$\beta_{ser17}$:

IFN-$\beta_{ser17}$ was recovered from E.coli that had been transformed to produce IFN-$\beta_{ser17}$. The E.coli were grown in the following growth medium to an OD of 10-11 at 680 nm (dry wt 8.4 g/l).

| Ingredient | Concentration |
|------------|---------------|
| $NH_4Cl$ | 20 mM |
| $K_2SO_4$ | 16.1 mM |
| $KH_2PO_4$ | 7.8 mM |
| $Na_2HPO_4$ | 12.2 mM |
| $MgSO_4 \cdot 7H_2O$ | 3 mM |
| $Na_3$ citrate $\cdot 2H_2O$ | 1.5 mM |
| $MnSO_4 \cdot 4H_2O$ | 30 μM |
| $ZnSO_4 \cdot 7H_2O$ | 30 μM |
| $CuSO_4 \cdot 5H_2O$ | 3 μM |
| L-tryptophan | 70 mg/l |
| $FeSO_4 \cdot 7H_2O$ | 72 μM |
| thiamine $\cdot HCl$ | 20 mg/l |
| glucose | 40 g/l |

pH control with $NH_4OH$

A 9.9 1 (9.9 kg) harvest of the transformed E.coli was cooled to 20° C and concentrated by passing the harvest through a cross-flow filter at an average pressure drop of ˜110 kpa and steady-state filtrate flow rate of 260 ml/min until the filtrate weight was 8.8 k9. The concentrate (approximately one liter) was drained into a vessel and cooled to 15° C. The cells in the concentrate were then disrupted by passing the concentrate through a Manton-Gaulin homogenizer at 5°C, ~69,000 kpa. The homogenizer was washed with one liter phosphate buffered saline, pH 7.4 (PBS), and the wash was added to the disruptate to give a final volume of two liters. This volume was continuously centrifuged at 12000 x g at a 50 ml/min flow rate. The solid was separated from the supernatant and resuspended in four liters PBS containing 2% by wt SDS. This suspension was stirred at room temperature for 15 min after which there was no visible suspended material. The solution was then extracted with 2-butanol at a 1:1 2-butanol:solution volume ratio. The extraction was carried out in a liquid-liquid phase separator using a flow rate of 200 ml/min. The organic phase was then separated and evaporated to dryness to yield 21.3 g of protein. This was resuspended in distilled water at a 1:10 volume ratio.

The recovered product was assayed for human IFN-$\beta$ activity using an assay based on protection against viral cytopathic effect (CPE). The assay was made in microtiter plates. Fifty $\mu$l of minimum essential medium were charged into each well and 25 $\mu$l of the sample was placed in the first well and 1:3 volume dilutions were made serially into the following wells. Virus (vesicular stomatitus), cell (human fibroblast line GM-2767), and reference IFN-$\beta$ controls were included on each plate. The reference IFN-$\beta$ used was 100 units per ml. The plates were then irradiated with UV light for 10 min. After irradiation 100 $\mu$l of the cell suspension (1.2 x $10^5$ cells/ml) was added to each well and the trays were incubated for 18-24 hr. A virus solution at one plaque-forming unit per cell was added to each well except the cell control. The trays were then incubated until the virus control showed 100% CPE. This normally occurred 18-24 hr after adding the virus solution. Assay results were interpreted in relation to the location of the 50% CPE well of the reference IFN-$\beta$ control. From this point the titer of interferon for all samples on the plate was determined. The specific activity of the recovered product was determined to be 5 x $10^7$ U/mg.

Example 8

Acid Precipitation And Chromatographic Purification

The Process of Example 7 was repeated except that after extraction and separation of the aqueous and organic phases and mixing of the organic phase with PBS at a volume ratio of 3:1 the pH of the mixture was lowered to about 5 by addition of glacial acetic acid. The resulting precipitate was separated by centrifugation at 10000-17000 x g for 15 min and the pellet was redissolved in 10% w/v SDS, 10 mM DTT, 50 mM sodium acetate buffer, pH 5.5, and heated to 80°C for 5 min.

The solution was then applied to a Brownlee RP-300, 10 $\mu$M, "Aquapore" column using a Beckman gradient system. Buffer A was 0.1% trifluoroacetic acid (TFA] in H$_2$O; buffer B was 0.1% TFA in acetonitrile. Detection was by ultraviolet absorbance at 280 nm. The solvent program was linear gradient of 0% buffer B to 100% buffer B in three hr. Fractions containing highest interferon activities were pooled and the specific activity of the pooled interferon preparation was determined to be 9.0 x $10^7$ to 3.8 X $10^8$ international units per mg protein, as compared to about 2 x $10^8$ U/mg for native IFN-$\beta$

Example 9

Biochemical Characterization of IFN-$\beta$ Ser $_{17}$

Amino acid compositions were determined after 24-72 hr timed hydrolysis of 40 $\mu$g samples of IFN in 200 $\mu$l of 5.7 N HCl, 0.1% phenol, at 108°C. Proline and cysteine were determined in the same fashion after performic acid oxidation; in this case, phenol was omitted from the hydrolysis. Tryptophan was analyzed after 24 hr hydrolysis of 400 $\mu$l samples in 5.7 N HCl, 10% mercaptoacetic acid (no phenol). Analysis was performed on a Beckman 121MB amino acid analyzer using a single column of AA10 resin. The amino acid composition calculated from representative 24-,48-, 72-hr acid hydrolyses of purified IFN-$\beta$ Ser$_{17}$ agrees well with that predicted by the DNA sequence of the clones IFN gene, minus the missing N-terminal methionine.

The amino acid sequence of the first 58 residues from the amino acid terminus of purified IFN was determined on a 0.7 mg sample in a Beckman 890C sequanator with 0.1 M Quadrol buffer. PTH amino acids were determined by reverse-phase HPLC on an Altex ultrasphere ODS column (4.6 x 250 mm) at 45°C eluted at 1.3 min at 40% buffer B, and 8.4 min from 40-70% buffer B, where buffer A was 0.0115 M sodium acetate, 5% tetrahydrofuran (THF), pH 5.11 and buffer B was 10% THF in acetonitrile.

The N-terminal amino acid sequence of IFN-$\beta$ Ser$_{17}$ determined matches the expected sequence predicted from the DNA sequence, except for the absence of N-terminal methionine.

As indicated above, the IFN-$\beta_{ser17}$ preparation exhibits specific activity levels very close to or better than that of native IFN-$\beta$. IFN-$\beta_{ser17}$ has no free sulfhydryl groups but indicates one -S-S- bond between the only remaining cysteines at positions 31 and 141. The protein does not readily form oligomers and appears to be substantially in the monomeric form. The IFN-$\beta_{ser17}$ may be formulated either as a single product or mixtures of the various forms, into pharmaceutically or veterinarily acceptable preparations, e.g. in inert, nontoxic, nonallergenic, physiologically compatible carrier media for clinical and therapeutic uses in cancer therapy or in conditions where interferon therapy is indicated and for viral infections. Such formulations may be in unit dosage form. Such media include but are not limited to distilled water, physiological saline, Ringer's solution, Hank's solution and the like. Other nontoxic stabilizing and solubilizing additives such as dextose, HSA (human serum albumin) and the like may be optimally included. The therapeutic formulations may be administered orally or parenterally such as intravenous, intramuscular, intraperitoneal and subcutaneous administrations. Preparations of the modified IFN-$\beta$ may also be used for topical applications in appropriate media normally utilized for such purposes.

The principal advantages of the above described mutein of IFN-$\beta$ lie in the elimination of a free sulfhydryl group at position 17 in IFN-$\beta$, thereby forcing the protein to form correct disulfide links between cys 31 and cys 141 and to assume the conformation ostensibly required for full biological activity. The increased specific activity of the IFN-$\beta_{ser17}$ enables the use of smaller dosages in therapeutic uses. By deleting the cysteine at position 17 and eliminating the free -SH group, the IFN-$\beta_{ser17}$ protein does not form dimers and oligomers so readily as the microbially produced IFN-$\beta$. This facilitates purification of the protein and enhances its stability.

Example 10

The nucleotide sequence for a cDNA clone coding for human IL-2, procedures for preparing IL-2 cDNA libraries, and screening same for IL-2 are described by Taniguchi, T., et al, Nature (1983) Vol 24, p 305 et seq.

cDNA libraries enriched in potential IL-2 cDNA clones were made from an IL-2 enriched mRNA fractions obtained from induced peripheral blood lymphocytes (PBL) and Jurkat cells by conventional procedures. The enrichment of the mRNA for IL-2 message was made by fractionating the mRNA and identifying the fraction having IL-2 mRNA activity by injecting the fractions in Xenopus laevis oocytes and assaying the oocyte lysates for IL-2 activity on HT-2 cells (J. Watson, J Exp Med (1979) 150 :1570-1519 and S. Gillis et al, J Immun (1978) 120: 2027-2032.)

Example 11

Screening and Identification of IL-2 cDNA Clones

The IL-2 cDNA libraries were screened using the colony hybridization procedure. Each microtiter plate was replicated onto duplicate nitrocellulose filter papers (S & S type BA-B5) and colonies were allowed to grow at 37° C for 14-16 hr on L agar containing 50 $\mu$g/ml ampicillin. The colonies were lysed and DNA fixed to the filter by sequential treatment for 5 min with 500 mM naOH, 1.5 M NaCl, washed twice for 5 min each time with 5 x standard saline citrate (SSC). Filters were air dried and baked at 80° C for 2 hr. The duplicate filters were pre-hybridized at 42° C for 6-8 hr with 10 ml per filter of DNA hybridization buffer (50% formamide, 5 x SSC, pH 7.0, 5 x Denhardt's solution (polyvinylpyrrolidine, plus ficoll and bovine serum albumin; 1 x = 0.2% of each), 50 mM sodium phosphate buffer at pH 7.0, 0.2% SDS, 20 $\mu$g/ml Poly U, and 5Q $\mu$g/ml denatured salmon sperm DNA.

A $^{32}$P-labeled 20-mer oligonucleotide probe was prepared based on the IL-2 gene sequence reported by Taniguchi, T., et al, supra. The nucleotide sequence of the probe was GTGGCCTTCTTGGGCATGTA.

The samples were hybridized at 42° C for 24-36 hr with 5 ml/filter of DNA hybridization buffer containing the $^{32}$P cDNA probe. The filters were washed two times for 30 min each time at 50° C with 2 x SSC, 0.1% SDS, then washed twice with 1 x SSC and 0.1% SDS at 50° C for 90 min, air dried, and autoradiographed at -70° C for 2 to 3 days. Positive clones were identified and rescreened with the probe.

Full length clones were identified and confirmed by restriction enzyme mapping and comparison with the sequence of the IL-2 cDNA clone reported by Taniguchi, T., et al, supra.

Example 12

Cloning of 11-2 Gene into M13 Vector

The IL-2 gene was cloned into M13mp9 as described in Example 1 using the plasmid pLW1 (Figure 12) containing the IL-2 gene under the control of the E.coli trp promoter. A sample of pLW1 was deposited in the American Type Culture Collection, 12301 Parklawn Drive, Rockville, Maryland 20852, USA, on 4 August 1983 and has been assigned ATCC number 39,405. me restriction map of one clone (designated M13-IL2) containing the IL-2 insert is shown in Figure 13. Single-stranded phage DNA was prepared from clone M13-IL2 to serve as a template for oligonucleotide-directed mutagenesis.

Example 13

Oligonucleotide-directed Mutagenesis

As indicated previously, IL-I contains cysteine residues at amino acid positions 58, 105 and 125. Based on the nucleotide sequences of the portions of the IL-2 gene that contain the codons for these three cysteine residues three oligonucleotide primers were designed and synthesized for mutating the codons for these residues to codons for serine. These oligonucleotides have the following sequences.

CTTCTAGAGACTGC AGA TGTTTC (DM27) to change cys 58,
CATCAGCATACTC AGA CATGAATG (DM28) to change cys 105 and
GATGATGCTCTG AGA AAAGGTAATC (DM29) to change cys 125.

Forty picomoles of each oligonucleotide were kinased separately in the presence of 0.1 mM ATP, 50 mM Tris-HCl, pH 8.0, 10 mM MgCl₂, 5 mM DTT and 9 units of T₄ kinase in 50 μl at 37°C for 1 hr. Each of the kinased primers (10 pmoles) was hybridized to 2.6 μg of ss M13-IL2 DNA in 15 μl of a mixture containing 100 mM NaCl, 20 mM Tris-HCl, pH 7.9, 20 mM MgCl₂ and 20 mM β-mercaptoethanol, by heating at 67°C for 5 min and 42°C for 25 min. The annealed mixtures were chilled on ice and then adjusted to a final colume of 25 μl of a reaction mixture containing 0.5 mM of each dNTP, 17 mM Tris-HCl, pH 7.9, 17 mM MgCl₂, 83 mM NaCl, 17 mM β-mercaptoethanol, 5 units of DNA polymerase I Klenow fragment, 0.5 mM ATP and 2 units of T₄ DNA ligase, incubated at 37°C for 5 hr. The reactions were terminated by heating to 80°C and the reaction mixtures used to transform competent JM103 cells, plated onto agar plates and incubated overnight to obtain phage placques.

Example 14

Screening and Identification of Mutagenized Phage Placques

Plates containing mutagenized M13-IL2 placques as well as 2 plates containing unmutagenized M13-IL2 phage placques, were chilled to 4°C and phage placques from each plate were transferred onto two nitrocellulose filter circles by layering a dry filter on the agar plate for 5 min for the first filter and 15 min for the second filter. The filters were then placed on thick filter papers soaked in 0.2 N NaOH, 1.5 M NaCl and 0.2% Triton for 5 min, and neutralized by layering onto filter papers soaked with 0.5 M Tris-HCl, pH 7.5, and 1.5 M NaCl for another 5 min. The filters were washed in a similar fashion twice on filters soaked in 2 x SSC, dried and then baked in a vacuum oven at 80°C for 2 hr. The duplicate filters were pre-hybridized at 42°C for 4 hr with 10 ml per filter of DNA hybridization buffer (5 x SSC, pH 7.0, 4 x Denhardts solution (polyvinylpyrrolidine, ficoll and bovin serum albumin, 1x = 0.02% of each), 0.1% SDS, 50 mM sodium phosphate buffer, pH 7.0 and 100 μg/ml of denatured salmon sperm DNA. ³²P-labelled probes were prepared by kinasing the oligonucleotide primers with labelled ATP. The filters were hybridized to 0.1 x 10⁵ cpm/ml of ³²P-labelled primers in 5 ml per filter of DNA hybridization buffer at 42°C for 8 hr. The filters were washed twice at 50°C for 30 min each in washing buffers containing 0.1% SDS and 2 x SSC, and twice at 50°C for 30 min each with 0.1% SDS and 0.2 x SSC. The filters were air dried and autoradiographed at -70°C for 2-3 days.

Since the oligonucleotide primers DM28 and DM29 were designed to create a new DdeI restriction site in the mutagenized clones (Figure 14), RF-DNA from a number of the clones which hybridized with each of these kinased primers were digested with the restriction enzyme DdeI . One of the mutagenized M13-IL2 placques which hybridized with the primer DM28 and has a new DdeI restriction site (M13-LW44) was picked and inoculated into a culture of JM103, ssDNA was prepared from the culture supernatant and dsRF-DNA was prepared from the cell pellet. Similarly, a placque which hybridized with primer DM29 was picked (M13-LW46) and ssDNA and RF-DNA prepared from it. The oligonucleotide primer DM27 was designed to create a new Pst I restriction site instead of a DdeI site. Therefore, the placques that hybridized to this primer were screened for the presence of a new PstI site. One such phage placque was identified (M13-LW42) and ssDNA and RF-DNA prepared from it. The DNA from all three of these clones were sequenced to confirm that the target TGT codons for cysteine had been converted to a TCT codon for serine.

Example 15

Recloning of the Mutagenized IL-2 Gene for Expression in E.coli

RF-DNA from M13-LW42, M13-LW44 and M13-LW46 were each digested with restriction enzymes Hind III and Ban II and the insert fragments purified from a 1% agarose gel. Similarly, the plasmid pTrp3 (Figure 7) was digested with Hind III and Ban II, the large plasmid fragment containing the trp promoter was purified on an agarose gel and then ligated with each of the insert fragments isolated from M13-LW42, M13-LW44 and M13-LW46. The ligated plasmids were transformed into competent E.coli K12 strain MM294. The plasmid DNAs from these transformants were analysized by restriction enzyme mapping to confirm the presence of the plasmids pLW42, pLW44 and pLW46. Figure 14 is a restriction map of pLW46. When each of these individual clones were grown in the absence of tryptophane to induce the trp promoter and cell free extracts analyzed on SDS-polyacrylamide gels, all three clones, pLW42, pLW44 and pLW46, were shown to synthesize a 14.5 kd protein similar to that found in the positive control, pLW21, which has been demonstrated to synthesize a 14.4 kd IL-2 protein. When these same extracts were subjected to assay for IL-2 activity on mouse HT-2 cells, only clones pLW21 (positive control) and pLW46 had significant amounts of IL-2 activity (Table II below), indicating that cys 58 and cys 105 are necessary for biological activity and changing them to serines (pLW42 and pLW44 respectively) resulted in the loss of biological activity. Cys 125 on the other hand must not be necessary for biological activity because changing it to ser 125 (pLW46) did not affect the biological activity.

## Table II

| Clones | IL-2 Activity ($\mu$/ml) |
|---|---|
| pIL2-7 (negative control) | 1 |
| pLW21 (positive control) | 113,000 |
| pLW42 | 660 |
| pLW44 | 1,990 |
| pLW46 | 123,000 |

Figure 15a shows the nucleotide sequence of the coding strand of clone pLW46. As compared to the coding strand of the native human IL-2 gene clone pLW46 has a single base change of G → C at nucleotide 374. Figure 15b shows the corresponding amino acid sequence of the IL-2 mutein encoded by pLW46. This mutein is designated IL-2$_{ser125}$ As compared to native IL-2 the mutein has a serine instead of a cysteine at position 125.

A sample of E.coli K12 strain MM294 transformed with pLW46 was deposited in the American Type Culture Collection, 12301 Parklawn Drive, Rockville, Maryland 20852, USA on 26 September 1983 and has been assigned ATCC Number 39,452.

Muteins of IL-2 in which the cysteine at position 125 has been replaced with another amino acid, such as the mutein IL-2$_{ser125}$ retain IL-2 activity. They may, therefore, be formulated and used in the same manner as native IL-2. Accordingly, such IL-2 muteins are useful in the diagnosis and treatment of bacterial, viral, parasitic, protozoan and fungal infections; in manifestations of lymphokine or immunodeficiency; for reconstitution of normal immunofunction in aged humans and animals; in the development of diagnostic assays such as those employing enzyme amplification, radiolabelling, radioimaging, and other methods known in the art for monitoring IL-2 levels in the diseased state; for the promotion of T cell growth in vitro for therapeutic and diagnostic purposes for blocking receptor sites for lymphokines; and in various other therapeutic, diagnostic and research applications. The various therapeutic and diagnostic applications of human IL-2 have been investigated and reported in S.A. Rosenberg, E.A. Grimm, et al, A. Mazumder, et al, and E.A. Grimm and S.A. Rosenberg. IL-2 muteins may be used by themselves or in combination with other immunologically relevent B or T cells or other therapeutic agents. For therapeutic or diagnostic applications (whether for human or veterinary use), they may, for example, be formulated in nontoxic, nonallergenic, physiolocally compatable carrier media such as distilled water, Ringer's solution, Hank's solution, physiological saline and the like. Administrations of the IL-2 muteins to humans or animals may be oral or intraperitoneal or intramuscular or subcutaneous as deemed appropriate by the physician. Examples of relevant cells are B or T cells, natural killer cells, and the like and exemplary therapeutic reagents which may be used in combination with the polypeptides of this invention are the various interferons, especially gamma interferon, B cell growth factor, IL-1 and the like.

**Claims**

1. A recombinant, synthetic biologically active mutein of a biologically active protein normally having at least one cysteine residue that is free to form a disulfide link and is non-essential to said biological activity in which at least one of said cysteine residues has been replaced by a neutral amino acid; provided that said mutein is not a serine$_{125}$interleukin-2 mutein, an alanine$_{125}$-interleukin-2 mutein, a threonine$_{125}$interleukin-2 mutein, a glycine$_{125}$interleukin-2 mutein, a valine$_{125}$interleukin-2 mutein, a leucine$_{125}$-interleukin-2 mutein, an isoleucine$_{125}$interleukin-2 mutein, a histidine$_{125}$interleukin-2 mutein, a tyrosine$_{125}$interleukin-2 mutein, a phenylalanine$_{125}$interleukin-2 mutein, a tryptophan$_{125}$interleukin-2 mutein, a methionine$_{125}$interleukin-2 mutein, a mutein of interferon-αA in which the cysteine residue(s) at Position 1 and/or Position 98 has/have been replaced, a mutein of interferon-αC to L in which the cysteine residue(s) at Position 1 and/or Position 99 has/have been replaced, a mutein of interferon-αB in which the cysteine residue(s) at Position 1 and/or Position 100 has/have been replaced, a mutein of interferon-αD or of a hybrid interferon-α containing an amino acid sequence of interferon-αD comprising the cysteine residue at Position 87 and in which the said cysteine residue has been replaced, or a mutein of a hybrid interferon-α in which the cysteine residue(s) at Position 1 and/or Position 98, 99 or 100 (as the case may be) has/have been replaced.

2. A synthetic mutein as claimed in claim 1, wherein there is only one of said cysteine residues.

3. A synthetic mutein as claimed in claim 1 or claim 2, wherein said cysteine residue(s) is/are replaced by serine or threonine, or by glycine, alanine, valine, leucine, isoleucine, histidine, tyrosine, phenylalanine, tryptophan or methionine.

4. A synthetic mutein as claimed in any one of claims 1 to 3 and which is unglycosylated.

5. A DNA sequence encoding a synthetic mutein as defined in any one of claims 1 to 4.

6. An expression vector including a DNA sequence as defined in claim 5 in a position that permits expression thereof.

7. A host cell which has been transformed with an expression vector as defined in claim 6, or progeny thereof retaining the ability to express said DNA sequence.

EP 0 192 811 B1

8. E.coli which has been transformed with an expression vector as defined in claim 6, or progeny thereof retaining the ability to express said DNA sequence.

9. A method for making DNA sequence encoding a recombinant, synthetic biologically active mutein of a biologically active protein normally having at least one cysteine residue that is free to form a disulfide link and is non-essential to said biological activity in which at least one of said cysteine residues has been replaced by a neutral amino acid; comprising:
(a) hybridizing single-stranded DNA comprising a DNA sequence encoding said protein with an oligonucleotide primer that is complementary to a region of said sequence that includes the codon for said at least one cysteine residue or the antisense triplet paired with said codon, as the case may be, except for a mismatch with said codon or said antisense triplet that defines a triplet that codes for a neutral amino acid;
(b) extending the primer with DNA polymerase to form a mutational heteroduplex; and
(c) replicating said mutational heteroduplex.

10. A method as claimed in claim 9, wherein the mismatch defines a triplet that codes for serine or threonine.

11. A method as claimed in claim 9 or claim 10, wherein the single-stranded DNA is provided by a single-stranded phage that includes said strand and the mutational heteroduplex of step (b) is converted to a closed circular heteroduplex.

12. A method as claimed in claim 11, wherein said replicating is effected by transforming a competent bacterial host with the closed circular heteroduplex and culturing the resulting transformants.

13. A method as claimed in any one of claims 9 to 12 also including the additional steps of isolating progeny of the mutant strand of the heteroduplex, isolating DNA from said progeny, and isolating said gene from the DNA from said progeny.

14. An oligonucleotide suitable for use in a method as defined in claim 9 and having a nucleotide sequence that is complementary to a region of the DNA sequence that includes the codon for the cysteine residue or the antisense triplet paired with said codon, as the case may be, except for a mismatch with said codon that defines a triplet that codes for a neutral amino acid.

15. A process for preparing an oligonucleotide suitable for use in a method as defined in claim 9 which comprises synthesizing by a manner known per se a nucleotide sequence that is complementary to a region of the DNA sequence that includes the codon for the cysteine residue or the antisense triplet paired with said codon, as the case may be, except for a mismatch with said codon that defines a triplet that codes for a neutral amino acid.

16. A process for producing a vector as defined in claim 6 comprising inserting into a vector capable of transforming a host cell a DNA sequence as defined in claim 5 in a position permitting expression of said sequence.

17. A process for producing a cell which is capable of the production of a mutein as defined in any one of claims 1 to 4, which process comprises transforming a cell lacking such capability as aforesaid with an expression vector including a DNA sequence encoding said mutein in a position that permits expression thereof.

18. A process for making a synthetic mutein as claimed in any one of claims 1 to 4, comprising culturing a host cell as defined in claim 7 or E.coli as defined in claim 8, or progeny thereof retaining the ability to express said DNA sequence, and harvesting the synthetic mutein from the resulting culture.

19. A method of preventing a protein having at least one cysteine residue that is free to form a disulfide link from forming said link comprising mutationally altering the protein to bring about replacement of the cysteine residue with a neutral amino acid, e.g., with serine or threonine; provided that the resulting mutationally altered protein is not a serine$_{125}$interleukin-2 mutein, an alanine$_{125}$-interleukin-2 mutein, a threonine$_{125}$interleukin-2 mutein, a glycine$_{125}$interleukin-2 mutein, a valine$_{125}$interleukin-2 mutein, a

16

leucine$_{125}$-interleukin-2 mutein, an isoleucine$_{125}$interleukin-2 mutein, a histidine$_{125}$interleukin-2 mutein, a tyrosine$_{125}$interleukin-2 mutein, a phenylalanine$_{125}$interleukin-2 mutein, a tryptophan$_{125}$interleukin-2 mutein, a methionine$_{125}$interleukin-2 mutein, a mutein of interferon-αA in which the cysteine residue(s) at Position 1 and/or Position 98 has/have been replaced, a mutein of interferon-αC to L in which the cysteine residue(s) at Position 1 and/or Position 99 has/have been replaced, a mutein of interferon-αB in which the cysteine residue(s) at Position 1 and/or Position 100 has/have been replaced, a mutein of interferon-αD or of a hybrid interferon-α containing an amino acid sequence of interferon-αD comprising the cysteine residue at Position 87 and in which the said cysteine residue has been replaced, or a mutein of a hybrid interferon-α in which the cysteine residue(s) at Position 1 and/or Position 98, 99 or 100 (as the case may be) has/have been replaced.

20. A method as claimed in claim 19, wherein the protein is biologically active and the cysteine is not essential to said biological activity.

21. A method of making a pharmaceutical or veterinary formulation, which method comprises formulating for pharmaceutical or veterinary use, respectively, a synthetic mutein as defined in any one of claims 1 to 4.

22. A pharmaceutical or veterinary formulation comprising a synthetic mutein as claimed in any one of claims 1 to 4 formulated for pharmaceutical or veterinary use, respectively, and optionally also comprising a pharmaceutically acceptable or veterinarily acceptable diluent, carrier or excipient and/or optionally being in unit dosage form.

23. A method of preventing a protein having at least one cysteine residue that is free to form a disulfide link from forming said link in a case where said link can be inhibitory of biological activity in said protein, comprising employing site-specific mutagenesis to produce a vector encoding said protein modified by said cysteine residue being replaced, cloning said vector using it to transform a host, using the resulting transformed hosts or progeny thereof retaining the ability to express said modified protein to express protein encoded by the genotype thereof, assessing proteins thus expressed by comparative biological effectiveness analysis to determine the clone corresponding to the desired modified protein, and using said clone to produce said protein as a biologically effective entity.

24. A synthetic mutein as claimed in any one of claims 1 to 4 for use in prophylaxis, therapy or diagnosis practised on the human or animal body.

25. The use of a synthetic mutein as claimed in any one of claims 1 to 4 in the preparation of a medicament.

## Revendications

1. Mutéine synthétique recombinante biologiquement active, dérivée d'une protéine biologiquement active comportant normalement au moins un résidu cystéine libre de former une liaison disulfure et non essentiel à l'activité biologique, dans laquelle au moins l'un des résidus cystéine a été remplacé par un aminoacide neutre ; à condition que cette mutéine ne soit pas une sérine$_{125}$interleukine-2 mutéine, une alanine$_{125}$-interleukine-2 mutéine, une thréonine$_{125}$-interleukine-2 mutéine, une glycine$_{125}$interleukine-2 mutéine, une valine$_{125}$interleukine-2 mutéine, une leucine$_{125}$interleukine-2 mutéine, une isoleucine$_{125}$interleukine-2 mutéine, une histidine$_{125}$interleukine-2 mutéine, une tyrosine$_{125}$interleukine-2 mutéine, une phénylalanine$_{125}$interleukine-2 mutéine, une tryptophane$_{125}$interleukine-2 mutéine, une méthionine$_{125}$-interleukine-2 mutéine, une mutéine de l'interféron-αA dans lequel le ou chaque résidu cystéine à la position 1 et/ou à la position 98, a été remplacé, une mutéine de l'interféron-αC à L dans lequel le ou chaque résidu cystéine à la position 1 et/ou à la position 99 a été remplacé, une mutéine de l'interféron-αB dans lequel le ou chaque résidu cystéine à la position 1 et/ou à la position 100 a été remplacé, une mutéine de l'interféron -αD ou d'un interféron-α hybride contenant une séquence d'aminoacides de l'interféron-αD comprenant le résidu cystéine à la position 87, et dans lequel ce résidu cystéine a été remplacé, ou une mutéine d'un interféron-α hybride dans lequel le ou chaque résidu cystéine à la position 1 et/ou à la position 98, 99 ou 100 (ainsi que cela peut être le cas) a été remplacé.

2. Mutéine synthétique selon la revendication 1, dans laquelle un seul desdits résidus cystéine est présent.

3. Mutéine synthétique selon la revendication 1 ou la revendication 2, dans laquelle le ou chaque résidu cystéine est remplacé par la sérine ou la thréonine, ou par la glycine, l'alanine, la valine, la leucine, l'isoleucine, l'histidine, la tyrosine, la phénylalanine, le tryptophane ou la méthionine.

4. Mutéine synthétique selon l'une quelconque des revendications 1 à 3, qui est non glycosylée.

5. Séquence d'ADN codant pour une mutéine synthétique selon l'une quelconque des revendications 1 à 4.

6. Vecteur d'expression comprenant une séquence d'ADN selon la revendication 5, dans une position qui permet son expression.

7. Cellule-hôte transformée avec un vecteur d'expression selon la revendication 6, ou la descendance de celui-ci conservant la capacité d'exprimer ladite séquence d'ADN.

8. E.coli transformé avec un vecteur d'expression selon la revendication 6, ou la descendance de celui-ci conservant la capacité d'exprimer ladite séquence d'ADN.

9. Procédé de préparation d'une séquence d'ADN codant pour une mutéine synthétique recombinante biologiquement active dérivée d'une protéine biologiquement active comportant normalement au moins un résidu cystéine libre de former une liaison disulfure et non essentiel à l'activité biologique, dans lequel au moins l'un de ces résidus cystéine a été remplacé par un aminoacide neutre ; selon lequel :
(a) on hybride un ADN monocaténaire comprenant une séquence d'ADN codant pour ladite protéine, avec une amorce oligonucléotidique complémentaire d'une région de cette séquence, qui comprend le codon correspondant à ceux ou à chaque résidu cystéine, ou le triplet anti-sens apparié à ce codon, ainsi que cela peut être le cas, à l'exception d'une erreur d'appariement avec ce codon ou ce triplet anti-sens, qui forme un triplet codant pour un aminoacide neutre ;
(b) on allonge l'amorce avec une ADN polymérase afin de former un hétéroduplex mutant ; et
(c) on procède à la réplication de cet hétéroduplex mutant.

10. Procédé selon la revendication 9, dans lequel l'erreur d'appariement forme un triplet qui code pour la sérine ou la thréonine.

11. Procédé selon la revendication 9 ou la revendication 10, dans lequel l'ADN à un seul brin est fourni par un phage monocaténaire comprenant ledit brin, et l'hétéroduplex mutant de l'étape (b) est converti en un hétéroduplex circulaire fermé.

12. Procédé selon la revendication 11, dans lequel la réplication est effectuée en transformant une bactérie-hôte compétente avec l'hétéroduplex circulaire fermé, et en cultivant les transformants résultants.

13. Procédé selon l'une quelconque des revendications 9 à 12, comprenant également les opérations supplémentaires consistant à isoler la descendance du brin mutant de l'hétéroduplex, à isoler l'ADN de cette descendance, et à isoler le gène de l'ADN issu de ladite descendance.

14. Oligonucléotide approprié pour être employé selon un procédé défini dans la revendication 9, et comportant une séquence de nucléotides complémentaire d'une région de la séquence d'ADN, comprenant le codon correspondant au résidu cystéine, ou le triplet anti-sens apparié à ce codon, ainsi que cela peut être le cas, à l'exception d'une erreur d'appariement d'appariement avec ce codon qui forme un triplet codant pour un aminoacide neutre.

15. Procédé de préparation d'un oligonucléotide approprié pour être employé selon un procédé défini dans la revendication 9, dans lequel on synthétise de façon connue en soi, une séquence de nucléotides complémentaire d'une région de la séquence d'ADN qui comprend le codon correspondant au résidu cystéine, ou le triplet anti-sens apparié à ce codon ainsi que cela peut être le cas, à l'exception d'une erreur d'appariement avec ce codon qui forme un triplet qui code pour un aminoacide neutre.

16. Procédé de préparation d'un vecteur selon la revendication 6, dans lequel on insert dans un vecteur capable de transformer une cellule-hôte, une séquence d'ADN définie dans la revendication 5, dans une position permettant l'expression de cette séquence.

17. Procédé de préparation d'une cellule capable de produire une mutéine selon l'une quelconque des revendications 1 à 4, dans lequel on transforme une cellule n'ayant pas la capacité précitée, avec un vecteur d'expression comprenant une séquence d'ADN codant pour ladite mutéine, dans une position qui permet son expression.

18. Procédé de préparation d'une mutéine synthétique selon l'une quelconque des revendications 1 à 4, dans lequel on cultive une cellule-hôte définie dans la revendication 7, ou E.coli défini dans la revendication 8, ou leur descendance conservant la capacité d'exprimer cette séquence d'ADN, et on recueille la mutéine synthétique à partir de la culture résultante.

19. Procédé pour empêcher une protéine comportant au moins un résidu cystéine libre de former une liaison disulfure, de former cette liaison, dans lequel on modifie par mutation la protéine en remplaçant 1e résidu cystéine par un aminoacide neutre, par exemple la sérine ou la thréonine ; à condition que la protéine modifiée par mutation résultante, ne soit pas une sérine$_{125}$interleukine-2 mutéine, une alanine$_{125}$interleukine-2 mutéine, une thréonine$_{125}$-interleukine-2 mutéine, une glycine$_{125}$interleukine-2 mutéine, une valine$_{125}$interleukine-2 mutéine, une leucine$_{125}$interleukine-2 mutéine, une isoleucine$_{125}$-interleukine-2 mutéine, une histidine$_{125}$interleukine-2 mutéine, une tyrosine$_{125}$interleukine-2 mutéine, une phénylalanine$_{125}$interleukine-2 mutéine, une tryptophane$_{125}$interleukine-2 mutéine, une méthionine$_{125}$-interleukine-2 mutéine, une mutéine de l'interféron-αA dans lequel le ou chaque résidu cystéine à la position 1 ou à la position 98 a été remplacé, une mutéine de l'interféron-αC à L dans lequel le ou chaque résidu cystéine à la position 1 ou à la position 99 a été remplacé, une mutéine de l'interféron-αB dans lequel le ou chaque résidu cystéine à la position 1 ou à la position 100 a été remplacé, une mutéine de l'interféron-αD ou d'un interféron-α hybride contenant une séquence d'aminoacides de l'interféron-αD comprenant le résidu cystéine à la position 87 et dans lequel ce résidu cystéine a été remplacé, ou une mutéine d'un interféron-α hybride dans lequel le ou chaque résidu cystéine à la position 1 ou à la position 98, 99 ou 100 (ainsi que cela peut être le cas) a été remplacé.

20. Procédé selon la revendication 19, dans lequel la protéine est biologiquement active et dans lequel la cystéine n'est pas essentielle à cette activité biologique.

21. Procédé de préparation d'une composition pharmaceutique ou vétérinaire, selon lequel on prépare respectivement pour une utilisation pharmaceutique ou vétérinaire, une mutéine synthétique définie dans l'une quelccnque des revendications 1 à 4.

22. Composition pharmaceutique ou vétérinaire comprenant une mutéine synthétique selon l'une quelconque des revendications 1 à 4 respectivement préparée pour une utilisation pharmaceutique ou vétérinaire, et comprenant également éventuellement un diluant, un véhicule ou un excipient acceptable au plan pharmaceutique ou vétérinaire, et/ou éventuellement sous une forme pharmaceutique unitaire.

23. Procédé pour empêcher une protéine comportant au moins un résidu cystéine libre de former une liaison disulfure, de former une telle liaison dans le cas ou cette liaison peut inhiber l'activité biologique de la protéine, selon lequel on effectue une mutagénèse spécifique à un site, pour produire un vecteur codant pour ladite protéine modifiée par remplacement du résidu cystéine, on clone ce vecteur en l'employant pour transformer un hôte, on emploie les hôtes transformés résultants ou leur descendance conservant la capacité de production de la protéine modifiée afin de produire la protéine codée par son génotype, on analyse les protéines ainsi produites par un essai comparatif de l'efficacité biologique afin de déterminer le clone correspondant à la protéine modifiée requise, et on emploie ce clone pour produire la protéine en tant que composé biologiquement actif.

24. Mutéine synthétique selon l'une quelconque des revendications 1 à 4, destinée à être employée pour effectuer une prophylaxie, une thérapie ou un diagnostic dans l'organisme humain ou animal.

**25.** Utilisation d'une mutéine synthétique selon l'une quelconque des revendications 1 à 4, pour préparer un médicament.

## Ansprüche

**1.** Rekombinantes, synthetisches, biologisch aktives Mutein eines biologisch aktiven Proteins mit normalerweise mindestens einem Cysteinrest, der frei ist und so eine Disulfidbrücke bilden kann und nicht-essentiell für die biologische Aktivität ist, in dem mindestens ein Cysteinrest durch eine neutrale Aminosäure ersetzt worden ist; Vorausgesetzt, daß das Mutein kein $Serin_{125}$-Interleukin-2-Mutein, $Alanin_{125}$-Interleukin-2-Mutein, $Threonin_{125}$-Interleukin-2-Mutein, $Glycin_{125}$-Interleukin-2-Mutein, $Valin_{125}$-Interleukin-2-Mutein, $Leucin_{125}$-Interleukin-2-Mutein, $Isoleucin_{125}$-Interleukin-2-Mutein, $Histidin_{125}$-Interleukin-2-Mutein, $Thyrosin_{125}$-Interleukin-2-Mutein, $Phenylalanin_{125}$-Interleukin-2-Mutein, $Tryptophan_{125}$-Interleukin-2-Mutein, $Methionin_{125}$-Interleukin-2-Mutein, Interferon-αA-Mutein, in dem der Cysteinrest bzw. die Cysteinreste in Position 1 und/oder Position 98 ersetzt, worden ist(sind), kein Mutein von Interferon-αC bis L, in dem der Cysteinrest bzw. die Cysteinreste in Position 1 und/oder Position 99 ersetzt worden ist (sind), Interferon-αB-Mutein, in dem der Cysteinrest bzw. die Cysteinreste in Position 1 und/oder Position 100 ersetzt worden ist (sind), Interferon-αD oder ein Hybrid-Interferon-α, enthaltend eine Interferon-αD-Aminosäuresequenz mit dem Cysteinrest in Position 87 und in der der Cysteinrest ersetzt worden ist oder

Hybrid-Interferon-α-Mutein ist, in dem der Cysteinrest bzw. die Cysteinreste in Position 1 und/oder Position 98, 99 oder 100 (je nach Fall) ersetzt worden ist bzw. sind.

**2.** Synthetisches Mutein nach Anspruch 1, das nur einen Cysteinrest enthält.

**3.** Synthetisches Mutein nach Anspruch 1 oder 2, in dem der Cysteinrest bzw. die Cysteinreste durch Serin, Threonin, Glycin, Alanin, Valin, Leucin, Isoleucin, Histidin, Thyrosin, Phenylalanin, Tryptophan oder Methionin ersetzt worden ist (sind).

**4.** Synthetisches Mutein nach einem der Ansprüche 1 bis 3, das nicht glycosyliert ist.

**5.** DNA-Sequenz, die ein synthetisches Mutein nach einem der Ansprüche 1 bis 4 codiert.

**6.** Expressionsvektor, der eine DNA-Sequenz nach Anspruch 5 in einer Position enthält, die ihre Expression erlaubt.

**7.** Wirtszelle, die mit einem Expressionsvektor nach Anspruch 6 transformiert worden ist, oder deren Nachkommenschaft, die die Fähigkeit beibehält, die DNA-Sequenz zu exprimieren.

**8.** E.coli, transformiert mit einem Expressionsvektor nach Anspruch 6, oder dessen Nachkommenschaft, die die Fähigkeit beibehält, die DNA-Sequenz zu exprimieren.

**9.** Verfahren zur Herstellung einer DNA-Sequenz, die ein rekombinantes, synthetisches, biologisch aktives Mutein eines biologisch aktiven Proteins codiert, das normalerweise mindestens einen Cysteinrest hat, der frei ist und so eine Disulfidbrücke bilden kann und nicht-essentiell für die biologische Aktivität ist, in dem mindestens ein Cysteinrest durch eine neutrale Aminosäure ersetzt worden ist, wobei man

(a) einzelsträngige DNA, die eine das Protein codierende DNA-Sequenz enthält, mit einem Oligonucleotid-Primer hybridisiert, der komplementär zu einem Bereich der Sequenz ist, die, je nach Fall, das Codon für mindestens einen Cysteinrest oder das mit dem Codon gepaarte antisense-Triplett enthält, mit Ausnahme einer Fehlpaarung mit dem Codon oder dem antisense-Triplett, die ein eins neutrale Aminosäure codierendes Triplett definiert;

(b) den Primer mit einer DNA-Polymerase verlängert, um einen Mutations-Heteroduplex zu bilden; und

(c) den Mutations-Heteroduplex repliziert.

**10.** Verfahren nach Anspruch 9, wobei die Fehlpaarung ein Serin- oder Threonin-codierendes Triplett definiert.

11. Verfahren nach Anspruch 9 oder 10, wobei die einzelsträngige DNA von einem Einzelstrang-Phagen bereitgestellt wird, der den Strang enthält, und der Mutations-Hetoroduplex aus Schritt (b) in einen geschlossenen zirkulären Heteroduplex umgewandelt wird.

12. Verfahren nach Anspruch 11, wobei die Replikation durch die Transformation eines kompetenten bakteriellen Wirts mit dem geschlossenen zirkulären Heteroduplex und der Züchtung der resultieranden Transformante bewirkt wird.

13. Verfahren nach einem der Ansprüche 9 bis 12, wobei man zusätzlich die Nachkommenschaft des mutierten Stranges des Heteroduplex, DNA aus der Nachkommenschaft und das Gen aus der DNA der Nachkommenschaft isoliert.

14. Oligonucleotid, geeignet zur Verwendung in einem verfahren nach Anspruch 9, mit einer Nucleotidsequenz, die komplementär zu einem Bereich der DNA-Sequenz ist, die, je nach Fall, das Codon für den Cystein-Rest oder das mit dem codon gepaarte antisense-Triplett enthält, mit Ausnahme einer Fehlpaarung mit dem Codon, die ein eine neutrale Aminosäure codierendes Triplett definiert.

15. Verfahren zur Herstellung eines Oligonucleotids, das zur Verwendung in einem Verfahren nach Anspruch 9 geeignet ist, wobei in einer an sich bekannten Weise eine Nucleotidsequenz synthetisiert wird, die komplementär zu einem Bereich der DNA-Sequenz ist, die, je nach Fall, das Codon für den Cysteinrest oder das mit dem Codon gepaarte antisense-Triplett enthält, mit Ausnahme einer Fehlpaarung mit dem Codon, die ein eine neutrale Aminosäure codierendes Triplett definiert.

16. Verfahren zur Herstellung eines Vektors nach Anspruch 6, wobei in einem zur Transformation einer Wirtszelle fähigen Vektor eine DNA-Sequenz nach Anspruch 5 in einer Position eingefügt wird, die ihre Expression erlaubt.

17. Vorfahren zur Herstellung einer Zelle, die die Fähigkeit zur Herstellung eines Muteins nach einem der Ansprüche 1 bis 4 besitzt, wobei eine Zelle, die diese Fähigkeit nicht besitzt, mit einem Expressionsvektor, der eine ein Mutein codierende DNA-Sequenz in einer Position enthält, die ihre Expression erlaubt, transformiert wird.

18. Verfahren zur Herstellung eines synthetischen Muteins nach einem der Ansprüche 1 bis 4, wobei man eine Wirtszelle nach Anspruch 7 oder E.coli nach Anspruch 8 oder deren Nachkommenschaft, die die Fähigkeit beibehält, die DNA-Sequenz zu exprimieren, züchtet, und das synthetische Mutein aus der so erhaltenen Kultur isoliert.

19. Verfahren zur Verhinderung der Bildung einer Brücke durch ein Protein mit mindestens einem Cysteinrest, der frei ist und so eine Disulfidbrücke bilden kann, wobei man das Protein durch Mutation so verändert, daß der Cysteinrest durch eine neutrale Aminosäure, z.B Serin oder Threonin, ersetzt wird; vorausgesetzt, daß das resultierende, durch Mutation veränderte Protein kein $Serin_{125}$-Interleukin-2-Mutein, $Alanin_{125}$-Interleukin-2-Mutein, $Threonin_{125}$-Interleukin-2-Mutein, $Glycin_{125}$-Interleukin-2-Mutein, $Valin_{125}$-Interleukin-2-Mutein, $Leucin_{125}$-Interleukin-2-Mutein, $Isoleucin_{125}$-Interleukin-2-Mutein, $Histidin_{125}$-Interleukin-2-Mutein, $Thyrosin_{125}$-Interleukin-2-Mutein, $Phenylalanin_{125}$-Interleukin-2-Mutein, $Tryptophan_{125}$-Interleukin-2-Mutein, $Methionin_{125}$-Interleukin-2-Mutein, Interferon-$\alpha$A-Mutein, in dem der Cysteinrest bzw. die Cysteinreste in Position 1 und/oder Position 98 ersetzt worden ist(sind), kein Mutein von Interferon-$\alpha$c bis L, in dem der Cysteinrest bzw. die Cysteinreste in Position 1 und/oder Position 99 ersetzt worden ist (sind), Interferon-$\alpha$B-Mutein, in dem der Cysteinrest bzw. die Cysteinreste in Position 1 und/oder Position 100 ersetzt worden ist (sind), Interferon-$\alpha$D-Mutein oder ein Hybrid-Interferon-$\alpha$, enthaltend eine Interferon-$\alpha$D-Aminosäuresequenz mit dem Cysteinrest in Position 87 und in der der Cysteinrest ersetzt worden ist oder Hybrid-Interferon-$\alpha$-Mutein ist, in dem der Cysteinrest bzw. die Cysteinreste in Position 1 und/oder Position 98, 99 oder 100 (je nach Fall) ersetzt worden ist bzw. sind.

20. Verfahren nach Anspruch 19, wobei das Protein biologisch aktiv und das Cystein für die biologische Aktivität nicht-essentiell ist.

21. Verfahren zur Herstellung einer pharmazeutischen oder veterinären Formulierung, wobei man synthetisches Mutein nach einem der Ansprüche 1 bis 4 zur pharmazeutischen oder veterinären Verwendung formuliert.

22. Pharmazeutische oder veterinäre Formulierung, die ein synthetisches Mutein nach einem der Ansprüche 1 bis 4, formuliert zur pharmazeutischen oder veterinären Verwendung und gegebenenfalls auch ein(en) pharmazeutisch oder veterinär verträgliches(en) Verdünnungsmittel, Träger oder Exzipient enthält und/oder gegebenenfalls als Einheitsdosierung vorliegt.

23. Verfahren zur Verhinderung der Bildung einer Brücke durch ein Protein mit mindestens einem Cysteinrest, der frei ist und so eine Disulfidbrücke bilden kann, in dem Fall, wo die Brücke die biologische Aktivität des Proteins hemmen kann, bei dem man Stellen-spezifische Mutagenese einsetzt, um einen Vektor herzustellen, der das durch Ersetzen des Cysteinrests veränderte Protein codiert, den Vektor cloniert, ihn zur Transformation eines Wirts verwendet, die resultierenden transformierten Wirtsorganismen oder deren Nachkommenschaft, die die Fähigkeit beibehält, das veränderte Protein zu exprimieren, zur Expression von Protein, das von deren Genotyp codiert wird, verwendet, die so exprimierten Proteine durch vergleichende Analyse der biologischen Wirksamkeit bewertet, um den Clon zu bestimmen, dar dem gewünschten veränderten Protein entspricht, und den Clon verwendet, um das Protein als biologisch wirksame Einheit herzustellen.

24. Synthetisches Mutein nach einem der Ansprüche 1 bis 4 zur Verwendung in der Prophylaxe, Therapie oder Diagnose, durchgeführt am menschlichen oder tierischen Kör-per.

25. Verwendung eines synthetischen Muteins nach einem der Ansprüche 1 bis 4 bei der Herstellung eines Arzneimittels.

MetSerTyrAsnLeu LeuGlyPheLeuGln ArgSerSerAsnPhe GlnCysGlnLysLeu
LeuTrpGlnLeuAsn GlyArgLeuGluTyr CysLeuLysAspArg MetAsnPheAspIle
ProGluGluIleLys GlnLeuGlnGlnPhe GlnLysGluAspAla AlaLeuThrIleTyr
GluMetLeuGlnAsn IlePheAlaIlePhe ArgGlnAspSerSer SerThrGlyTrpAsn
GluThrIleValGlu AsnLeuLeuAlaAsn ValTyrHisGlnIle AsnHisLeuLysThr
ValLeuGluGluLys LeuGluLysGluAsp PheThrArgGlyLys LeuMetSerSerLeu
HisLeuLysArgTyr TyrGlyArgIleLeu HisTyrLeuLysAla LysGluTyrSerHis
CysAlaTrpThrIle ValArgValGluIle LeuAgAsnPheTyr PheIleAsnArgLeu
ThrGlyTyrLeuArg Asn---

# FIG. I

primer
↓
5'-GCAATTTTCAG A GTCAG-3'
TCTTCGTCGTTAAAAGTC A CAGTCTTCGAGGAC

IFN-β

M13

DNA polymerase I repair
T₄ DNA ligase

transform *E. coli*

GCAATTTTCAG T GTCAG
CGTTAAAAGTC A CAGTC

*Hinf* I
↓
GCAATTTTCAG A GTCAG
CGTTAAAAGTC T CAGTC

IFN-β

IFN-β

M13 (RF)

M13 (RF)

Parent

Mutant

FIG. 2

FIG. 3

FIG. 4

FIG. 5

FIG. 7

FIG. 6

FIG. 8a

FIG. 8b

28

FIG. 9

IFN-B CYS TO SER CHANGE AT AMINO ACID 17

```
                                                         17
1
ATG AGC TAC AAC TTG CTT GGA TTC CTA CAA AGA AGC AGC AAT TTT CAG AGT CAG AAG CTC
met ser tyr asn leu leu gly phe leu gln arg ser ser asn phe gln ser gln lys leu

61
CTG TGG CAA TTG AAT GGG AGG CTT GAA TAT TGC CTC AAG GAC AGG ATG AAC TTT GAC ATC
leu trp gln leu asn gly arg leu glu tyr cys leu lys asp arg met asn phe asp ile

121
CCT GAG GAG ATT AAG CAG CTG CAG CAG TTC CAG AAG GAG GAC GCC GCA TTG ACC ATC TAT
pro glu glu ile lys gln leu gln gln phe gln lys glu asp ala ala leu thr ile tyr

181
GAG ATG CTC CAG AAC ATC TTT GCT ATT TTC AGA CAA GAT TCA TCT AGC ACT GGC TGG AAT
glu met leu gln asn ile phe ala ile phe arg gln asp ser ser ser thr gly trp asn

241
GAG ACT ATT GTT GAG AAC CTC CTG GCT AAT GTC TAT CAT CAG ATA AAC CAT CTG AAG ACA
glu thr ile val glu asn leu leu ala asn val tyr his gln ile asn his leu lys thr

301
GTC CTG GAA GAA AAA CTG GAG AAA GAA GAT TTC ACC AGG GGA AAA CTC ATG AGC AGT CTG
val leu glu glu lys leu glu lys glu asp phe thr arg gly lys leu met ser ser leu

361
CAC CTG AAA AGA TAT TAT GGG AGG ATT CTG CAT TAC CTG AAG GCC AAG GAG TAC AGT CAC
his leu lys arg tyr tyr gly arg ile leu his tyr leu lys ala lys glu tyr ser his

421
TGT GCC TGG ACC ATA GTC AGA GTG GAA ATC CTA AGG AAC TTT TAC TTC ATT AAC AGA CTT
cys ala trp thr ile val arg val glu ile leu arg asn phe tyr phe ile asn arg leu

481
ACA GGT TAC CTC CGA AAC TGA AGA TC
thr gly tyr leu arg asn ***
```

# FIG. 10

FIG. II

pSY2501   pβ1trp   ptrp3

93kd

66kd

45kd

31kd

21kd

IFNβ →

14kd

FIG. 12

FIG. 13

FIG. 14

```
        10            20          30          40          50          60
ATGCCTACTT  CAAGTTCTAC  AAAGAAAACA  CAGCTACAAC  TGGAGCATTT  ACTGCTGGAT
        70            80          90         100         110         120
TTACAGATGA  TTTTGAATGG  AATTAATAAT  TACAAGAATC  CCAAACTCAC  CAGGATGCTC
       130           140         150         160         170         180
ACATTTAAGT  TTTACATGCC  CAAGAAGGCC  ACAGAACTGA  AACATCTTCA  GTGTCTAGAA
       190           200         210         220         230         240
GAAGAACTCA  AACCTCTGGA  GGAAGTGCTA  AATTTAGCTC  AAAGCAAAAA  CTTTCACTTA
       250           260         270         280         290         300
AGACCCAGGG  ACTTAATCAG  CAATATCAAC  GTAATAGTTC  TGGAACTAAA  GGGATCTGAA
       310           320         330         340         350         360
ACAACATTCA  TGTGTGAATA  TGCTGATGAG  ACAGCAACCA  TTGTAGAATT  TCTGAACAGA
       370           380         390         400         410         420
TGGATTACCT  TTTCTCAGAG  CATCATCTCA  ACACTGACTT  GA
```

# FIG. 15a

```
        5                10               15               20
MetProThrSerSer  SerThrLysLysThr  GlnLeuGlnLeuGlu  HisLeuLeuLeuAsp
        25               30               35               40
LeuGlnMetIleLeu  AsnGlyIleAsnAsn  TyrLysAsnProLys  LeuThrArgMetLeu
        45               50               55               60
ThrPheLysPheTyr  MetProLysLysAla  ThrGluLeuLysHis  LeuGlnCysLeuGlu
        65               70               75               80
GluGluLeuLysPro  LeuGluGluValLeu  AsnLeuAlaGlnSer  LysAsnPheHisLeu
        85               90               95              100
ArgProArgAspLeu  IleSerAsnIleAsn  ValIleValLeuGlu  LeuLysGlySerGlu
       105              110              115              120
ThrThrPheMetCys  GluTyrAlaAspGlu  ThrAlaThrIleVal  GluPheLeuAsnArg
       125              130              135              140
TrpIleThrPheSer  GlnSerIleIleSer  ThrLeuThr---
```

# FIG. 15b

35